(19) Europäisches Patentamt
European Patent Office
Office européen des brevets

(11) EP 3 362 059 B1

(12) EUROPEAN PATENT SPECIFICATION

(45) Date of publication and mention of the grant of the patent:
**01.01.2020 Bulletin 2020/01**

(21) Application number: **16784165.9**

(22) Date of filing: **17.10.2016**

(51) Int Cl.:
***A61K 31/185*** (2006.01) ***A61K 31/7056*** (2006.01)
***A61K 9/00*** (2006.01) ***A61P 25/00*** (2006.01)

(86) International application number:
**PCT/EP2016/074828**

(87) International publication number:
**WO 2017/064317 (20.04.2017 Gazette 2017/16)**

(54) **NEW COMBINATION THERAPIES FOR TREATING NEUROLOGICAL DAMAGE**

NEUE KOMBINATIONSTHERAPIEN ZUR BEHANDLUNG NEUROLOGISCHER SCHÄDEN

NOUVELLES THÉRAPIES COMBINÉES POUR LE TRAITEMENT DE LÉSIONS NEUROLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.10.2015 EP 15382508**

(43) Date of publication of application:
**22.08.2018 Bulletin 2018/34**

(73) Proprietor: **Universitat Autònoma de Barcelona**
**08193 Bellaterra (Cerdanyola del Valles) (ES)**

(72) Inventors:
- **CASAS LOUZAO, Caty**
  **08193 Bellaterra (Cerdanyola) - Barcelona (ES)**
- **ROMEO GUITART, David**
  **08193 Bellaterra (Cerdanyola) - Barcelona (ES)**
- **FORÉS VIÑETA, Joaquim**
  **08193 Bellaterra (Cerdanyola) - Barcelona (ES)**
- **COMA, Mireia**
  **08007 Barcelona (ES)**
- **PUJOL, Albert**
  **08320 El Masnou - Barcelona (ES)**
- **MAS, José, Manuel**
  **08023 Barcelona (ES)**

(74) Representative: **ZBM Patents ApS**
**Symbion Box:33**
**Fruebjergvej 3**
**2100 Copenhagen Ø (DK)**

(56) References cited:
**WO-A1-01/45509**
**US-A1- 2010 216 734**
**US-A1- 2014 371 229**
**US-A1- 2014 378 440**
**US-B1- 6 680 292**

EP 3 362 059 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

## FIELD OF THE INVENTION

The present invention relates generally to the field of nerve damage. The invention features a drug combination which promotes neuroregeneration and neuroprotection against endoplasmic reticulum (ER) stress and is useful for the treatment and/or prevention of neurological damage, and particularly peripheral nerve injury. The invention also relates to pharmaceutical compositions and methods for the treatment and/or prevention of neurological damage, and particularly peripheral nerve injury and cranial nerve injury.

## BACKGROUND OF THE INVENTION

Traumatic injuries to the peripheral nerve system (PNS) are a major cause of disability worldwide, accounting for 2 to 3% of the cases in Level I trauma centers during peacetime, and many more in wartime. As a result of this high incidence, which entails considerable economic and social impact, the interest in this field has been steadily growing during the last years.

The peripheral nerves an extensive network of nerves that are the tool for the brain and spinal cord to communicate with the rest of the body. A nerve is an enclosed, bundle of afferent and efferent fibers to sensorial neurons or from motoneurons, respectively. The nerves are fragile and can be damaged easily. When one of these nerves suffers injury or trauma, surgical treatment is sometimes the only remedy. There are more than one hundred kinds of peripheral nerve disorders affecting one nerve or many nerves. Some are the result of other diseases, like diabetic nerve problems and other, like the autoimmune disease Guillain- Barre syndrome, happen after a virus infection. Still others are from nerve compression, like carpal tunnel syndrome and thoracic outlet syndrome, or appearing after injury such as complex regional pain syndrome and brachial plexus injury.

Spinal nerve roots can result compressed due to vertebral disc herniation and tumors, such as neurofibromas or meningeomas. High energy accidents involving traction of the upper limbs or strong neck lateralization may result in brachial plexus lesion. Such injuries lead to a variety of sensory and motor dysfunctions. In this situation, spinal roots may be avulsed, crushed and tractioned, resulting in a complex lesion with inflammatory reaction, Wallerian degeneration of transected nerves and which may lead to retrograde neurodegeneration, although sometimes, since the epi and perineural connective tissue remain preserved, the degree of injury is underestimated. Thus, neuronal loss is also expected after ventral root crushing such as the one produced by disc herniation, that may in turn reduce the success of certain surgical procedures such as nerve graft and end-to-side neurorrhaphy. Distal nerve compression (for instance after carpal tunnel syndrome) although is rarely leading to retrograde neurodegeneration, axons are damaged, and longer periods of compression may finally com promise the whole nerve system, neuronal function and survival.

After injuries that cause the rupture of peripheral nerve fibers, axonal degeneration takes place through two distinct mechanisms: Wallerian or orthograde degeneration, which occurs toward the distal axon, and retrograde degeneration, which progresses toward the proximal cell body While Wallerian degeneration attempts to create a favorable environment for the axonal regrowth in the distal side, retrograde degeneration promotes a series of phenotypic changes in the proximal segment and the initiation of a neuronal reaction. Axonal breakdown immediately produces rapid elevation of $Ca^{2+}$ and cAMP levels through damaged axon membranes, deprivation of retrogradely transported target-derived neurotrophins and loss of synaptic connectivity and neuronal activity necessary for survival. Disruption of the tight ionic concentration gradient through membrane leaks may causes activation of several cascade signaling pathways.

In contrast to the extensive loss of MNs after root avulsion (RA), a distal nerve axotomy (DA) will result in an anterograde axonal degeneration in the peripheral nervous system and parts of the central nervous system nerve fibers thereof, but rarely to neuronal death (see Valero-Cabré A, Peripheral and spinal motor reorganization after nerve injury and repair, J Neurotrauma. 2004). In order to shed light into this neurodegenerative process, in the last years, we have collected growing evidence showing that the molecular events activated after RA, although similar to those triggered after DA at initial days post operation (dpo), they started to diverge by 7 dpo characterized by the unbalanced response to pro-survival mechanisms such as the response to ER stress or autophagy (see Penas C, Autophagy, and BiP level decrease are early key events in retrograde degeneration of motoneurons, Cell Death Differ. 2011).

The fact that human neurons, and more specifically motoneurons (MNs), inherently possess the ability to regenerate, suggests that therapies that enhance or optimize this innate capacity might be applicable. Neuroprotective strategies for MN survival after nerve injuries are nonexistent in the clinical practice. Several molecules have been tested in experimental models, which are summarized in table 1. Two main models are best known and used: spinal nerve or root avulsion, with or without reimplantation, and facial nerve avulsion. In this table, the drugs are organized in such a manner that the ones with higher reported neuroprotective effects for each type of model are at the top. Although riluzole has the maximum observed effect of neuroprotection in a model with reimplantation, in models of root avulsion without reimplantation it shows no neuroprotective effect (see Chew DJ, The effects of minocycline or riluzole treatment on

spinal root avulsion-induced pain in adult rats, J Pain. 2014). Among the best neuroprotective agents found were Pre084 with an induction of around 50-60% of MN survival (see Penas, C., et al. Sigma Receptor Agonist 2-(4-Morpholinethyl)1 Phenylcyclohexanecarboxylate (Pre084) Increases GDNF and BiP Expression and Promotes Neuroprotection after Root Avulsion Injury. J. Neurotrauma, 2011, 28, 831-840). In the facial nerve model, vitamin E and a combination of neurotrophins yielded neuroprotection of facial MNs up to 80 % but the latest led to collateral health problems at clinics.

Table 1. Drugs with some neuroprotective effect in experimental models of nerve avulsion

| *Drug* | *Model* |
|---|---|
| Riluzole | Spinal Root avulsion+reimplant(1) |
| Glatiramer acetate | Spinal Root avulsion |
| GM-1 ganglioside | Spinal Root avulsion |
| Pre084 | Spinal Root avulsion |
| Cerebrolysin | Spinal Root avulsion |
| Valproic acid | Spinal Root avulsion |
| Minocicline | Spinal Root avulsion |
| N-acetyl-cysteine | Spinal Root avulsion |
| Phorbol-12-myristate-13-acetate | Spinal Root avulsion |
| Vitamin E | Facial nerve avulsion |
| BDNF+GDNF | Facial nerve avulsion |
| T-588 (derivative of acetylcholine) | Facial nerve avulsion |
| NOS inhibitor | Facial nerve avulsion |
| Ro5-4864 | Facial nerve avulsion |
| (1) Note that reimplantation at the moment of the avulsion gives extra-benefits in terms of neuroprotection but in clinical scenarios it is impossible to carry it out due to the need for patient stabilization after damage. | |

In addition of MN neuroprotection, in order to reconstitute the nervous function loss by axonal injuries, two other factors should be taken into consideration: the prevention of neuropathic pain for which there is no treatment, and the promotion of axonal regeneration. Neuroregeneration, consists of the regrowth or repair of nervous tissues, cells or cell products through the generation of new neurons, glia, axons, myelin, or synapses.

The key mechanisms that determine the outcome of this process are not yet fully characterized. However, among several factors needed, the success of regeneration and functional reinnervation depend on the capacity of the damaged neurons to survive and shift to a regenerative phenotype (see Navarro X, Neural plasticity after peripheral nerve injury and regeneration, Prog Neurobiol. 2007). Due to the multitude of physiological pathways that mediate both cell death and its subsequent regeneration, it is becoming increasingly clear that multi-target polypharmacological research is needed to interact with different targets and modify different molecular pathways. Clinical success with multicomponent therapies and multi-targeted agents has been shown in other pathologies like asthma (fluticasone and propionate/salmeterol), hyperlipidemia (Lovastatin and extended-release niacin combination), HIV-1 (AZT-3TC) or cancer.

US2010216734 describes compositions comprising a neurogenic or neurogenic sensitizing agent, such as acamprosate or ribavirin, in combination with a nootropic agent for use in the treatment of neurological damages.

A large number of neurological disorders are considered to be associated with motoneuron damage, and particularly with ER stress. ER stress is induced by accumulation of unfolded protein aggregates or by excessive protein traffic, leading to the overload of the protein synthesis machinery. The accumulation of these aggregates leads to cell death through various mechanisms such as PERK, IRE-1 and ATF-6. Diseases and disorder in this group may include efferent fiber lesions such as polyneuropathies, nerve entrapments, nerve compression syndrome (compressed neuropathy) and nerve traumatism; lesions of the posterior (dorsal) root of the spinal cord like radiculopathies, spinal disc herniations, tumours and radiotherapy; lesions of the motoneurons of the posterior (ventral) root of the spinal cord including acute polyomelitis; and diseases affecting lower MNs like peripheral neuropathies, amyotrophic lateral sclerosis (ALS), spinal muscular atrophy (SMA) (see Montague K, Endoplasmic reticulum stress in spinal and bulbar muscular atrophy: a potential target for therapy, Brain. 2014). Moreover, pro-regenerative treatments could be useful for prevention

of inflammatory and neurodegenerative processes ensuing after the implantation of surgical prostheses.

Furthermore, ER stress is associated with a wide range of neurodegenerative diseases (see Hetz C, Disturbance of endoplasmic reticulum proteostasis in neurodegenerative diseases, Nat Rev Neurosci. 2014) such as amyotrophic lateral sclerosis, and other that are not linked to motoneuron death: Huntington's disease, Creutzfeldt-Jakob disease, Alzheimer's disease, frontotemporal dementia, Parkinson's disease among others.

There is a need for new treatments of neurological damage, such as peripheral nerve injury. In particular, there is no treatment to promote neuroregeneration and neuroprotection against endoplasmic reticulum (ER) stress.

## SUMMARY OF THE INVENTION

The inventors have found that a combination of Acamprosate and Ribavirin (C1) is capable of promoting neuroprotection of degenerating neurons, *in vivo. In vitro,* C1 treatment is capable to promote neuroprotection against ER stress. Besides, C1 treatment is capable to promote regenerative profile in neurons and reduce inflammation of the neural tissue by reducing gliosis. The inventors have also found that one of the components involved in the mechanism of action of C1 was through the activation of sirtuin 1 (SIRT1). Sirtuins have been demonstrated to be involved in promoting life-span extension and increasing their activity may protect in front of several pathologies such as metabolic disorders, neurodegeneration and inflammation.

Thus, the present invention is directed to a combination of Acamprosate and Ribavirin, to pharmaceutical compositions, kits and methods for the treatment and/or prevention of neurological damage, particularly peripheral nerve lesion, and other lesions of efferent/afferent fibers such as polyneuropathies, nerve entrapments and nerve compression syndrome (such as carpal tunnel syndrome, meralgia paresthetica, thoracic outlet syndrome, ulnar nerve entrapment, compressed neuropathy) and nerve traumatism (such as brachial plexus injury, foot drop injury, spinal accessory nerve injury, traumatic nerve injury, complex regional pain syndrome); lesions of the posterior (dorsal) root of the spinal cord like radiculopathies, spinal disc herniations, or caused by tumours (such as malignant nerve sheath tumors, neurofibromas, schwannomas) and radiotherapy; lesions or traumatic cranial neuropathy affecting cranial nerves (olfactory, oculomotor, trochlear, abducens, facial, vestibulocochlear, accessory and hypoglossal nerves, trigeminal nerve and ganglion, optic nerve and chiasm); diseases of the motoneurons of the posterior (ventral) root of the spinal cord including acute polyomelitis or motor axons such as Guillain-Barré syndrom; and diseases affecting lower motoneurons like peripheral neuropathies, amyotrophic lateral sclerosis (ALS), spinal muscular atrophy (SMA); acute and chronic inflammatory pain, neuropathic pain, neuritis, phantom limb pain, radiculopathy, traumatic injury. Moreover, the novel combinations are also useful for prevention of inflammatory and their associated neurodegenerative processes or painful states ensuing after the implantation of surgical prostheses.

In a first aspect the present invention is directed to a combination comprising acamprosate or a pharmaceutically acceptable salt thereof, and ribavirin or a pharmaceutically acceptable salt thereof.

In a second aspect the present invention is directed to a pharmaceutical composition comprising a combination which comprises acamprosate or a pharmaceutically acceptable salt thereof, and ribavirin or a pharmaceutically acceptable salt thereof.

In a third aspect the present invention is directed to a combination comprising acamprosate or a pharmaceutically acceptable salt thereof, and ribavirin or a pharmaceutically acceptable salt thereof, or to a pharmaceutical composition comprising said combination for use in medicine.

In a fourth aspect the present invention is directed to a combination comprising acamprosate or a pharmaceutically acceptable salt thereof, and ribavirin or a pharmaceutically acceptable salt thereof, or to a pharmaceutical composition comprising said combination for use in the treatment and/or prevention of neurological damage.

The combination comprising acamprosate or a pharmaceutically acceptable salt therof, and ribavirin or a pharmaceutically acceptable salt thereof, is particularly suitable for treating and/or preventing neurological damage, particularly peripheral nerve lesion, and other lesions of efferent/afferent fibers such as polyneuropathies, nerve entrapments and nerve compression syndrome (such as carpal tunnel syndrome, meralgia paresthetica, thoracic outlet syndrome, ulnar nerve entrapment, compressed neuropathy), and nerve traumatism (such as brachial plexus injury, foot drop injury, spinal accessory nerve injury, traumatic nerve injury, complex regional pain syndrome); lesions of the posterior (dorsal) root of the spinal cord like radiculopathies, spinal disc herniations, or caused by tumours (such as malignant nerve sheath tumors, neurofibromas, schwannomas) and radiotherapy; lesions or traumatic cranial neuropathy affecting cranial nerves (olfactory, oculomotor, trochlear, abducens, facial, vestibulocochlear, accessory and hypoglossal nerves, trigeminal nerve and ganglion, optic nerve and chiasm); diseases of the motoneurons of the posterior (ventral) root of the spinal cord including acute polyomelitis or motor axons such as Guillain-Barré syndrome; and diseases affecting lower motoneurons like peripheral neuropathies, amyotrophic lateral sclerosis (ALS), spinal muscular atrophy (SMA); acute and chronic inflammatory pain, neuropathic pain, neuritis, phantom limb pain, radiculopathy, traumatic injury. Moreover, the combination of the present invention is also useful for prevention of inflammatory and neurodegenerative processes ensuing after the implantation of surgical prostheses.

In a fifth aspect the present invention is directed to a kit comprising the combination comprising acamprosate or a pharmaceutically acceptable salt therof, and ribavirin or a pharmaceutically acceptable salt thereof, wherein acamprosate or a pharmaceutically acceptable salt thereof and ribavirin or a pharmaceutically acceptable salt thereof are disposed in separate containers.

**BRIEF DESCRIPTION OF THE DRAWINGS**

FIGURE 1. C1 promotes neuroprotection *in vivo. Top,* Draw of spinal cord framing the ipsilateral ventral horn analyzed in subsequent representative microphotographs. Photos of fluorescent nissl stained spinal cord ventral horns of control sham or the ipsilateral lesioned site at L4-L5 level. Rats were avulsed on their right side and intratechally treated with either vehicle aCSF, 1.7 μg/Kg/day PRE084 with the combination C1= 96 μg/Kg/day Acamprosate calcium (equivalent to 86.9 μg/Kg/day free acamprosate) + 0.48 μg/Kg/day Ribavirin. *Bottom,* Bar graph of the average of the relative percentage of the number of survival motoneurons +/- SEM at the ipsilateral side respect to the contralateral non-injured side after 21 days post-injury with or without the respective treatments. (n=3 for Sham, PRE084, n=6 for injured; n=4 for other groups, ANOVA, post hoc Bonferroni * p<0.05 respect to vehicle,). Scale bar = 100 μm.

FIGURE 2. C1 reduces gliosis at the central nervous system. *Left,* Representative microphotographs of fluorescent labeled astrocytes (*top panel,* GFAP labeling) or microglia at low and high magnification (*both middle panels,* Iba1 labeling), or pro-regenerative axons (*bottom panel,* GAP43 labelling) at spinal cord of the ipsilateral lesioned site at L4-L5 level. Rats were avulsed on their left site and intratechally treated with either vehicle aCSF, 1.7 μg/Kg/day PRE084 (P), or with the combination C1= 96 μg/Kg/day Acamprosate calcium (equivalent to 86.9 μg/Kg/day acamprosate free acid) + 0.48 μg/Kg/day Ribavirin. *Right,* Bar graph of the average of the percentage of immunofluorecent intensity (% IF intensity) within a fixed region of interest at the ipsilateral ventral horn respect to the contralateral side in different conditions. (n=3-6, ANOVA, post hoc Bonferroni *p<0.05 respect to vehicle,). Scale bar = 100μm.

FIGURE 3. C1 is neuroprotective to face ER stress and presented supra-addictive effects. A. Bar graph showing the average +/- SEM of the percentage of cell density in non-stressed NSC-34 cells when treated with C1 or each drug component alone at dose 0.11 mM Acamprosate calcium (ACA), 4 μM Ribavirin (RIB) within 100 μl final volume. B. Bar graph showing the average +/- SEM of the percentage of cell survival of ER stressed cells when treated with different concentrations of Tunycamicine (TN) (from 0.1 to 10 μg/ml within 100 μl final volume, or with concomitant treatment with 1 μg/ml TN and either the vehicle, C1 (2X, formed by 0.11 mM acamprosate calcium and :4 μM RIB) or single drugs (0.11 mM acamprosate calcium or :4 μM RIB, or 10 μM PRE084), analysed by an MTT assay (densitometry 562 nm readout) (n=3-8, *p<0.05 respect to vehicle, #p<0.05 respect to 1 μg/ml TN).

FIGURE 4. *In silico* predicted mode of action of C1 revealed Itgb1, Kif5c, and SIRT1 as targets. A. Microphotographs of MNs immunostained to reveal the present of each of these targets counterstained with green fluorescent Nissl in the L4-L5 spinal cord ventral horns of injured animals treated with either vehicle (Veh) or C1 (C, contralateral, I, ipsilateral). *Bottom,* bar graph of the average of the ratio of immunofluorescence intensity between contra and ipsilateral sides within a pre-determined region of interest (ROI) in the lateral gray matter at each condition. For SIRT1 analysis, MN nuclei were selected as ROI to compare immunofluorescent intensity (% IF Intens) in the ipsilateral sides of vehicle and C1 treated animals (n=4 animals, 5 MN/section, 3 sections, *p<0.05, scale bar= 50 μm). B. Images of DCTN1 staining in axonal traks at the ventral horn in animals treated by different conditions. *Bottom,* Bar graphs of the ratio of immunofluorescent intensity between ipsi and contralateral sides (*left*) or the integrate density (Int Dens) within a ROI at each side (*right*) to show the specificity of C1 for the analysed effect over a particular pathological condition.

FIGURE 5. C1 neuroprotection is mediated by SIRT1 activation. Representative microphotographs of Nissl stained ventral horns (*left*) and histogram (*right*) showing the average of the percentage +/- SEM of MN survival after different treatments with spermidine (S) or Ex-527 with or without C1 (n=4, ANOVA, post hoc Bonferroni *p<0.05 respect to untreated, # p<0.05 respect to veh, $ p<0.05 respect to C1+Ex527, *p<0.05). Scale bar = 100 μm.

**FIGURE 6. C1 accelerates nerve regeneration and improve muscle reinnervation and functional recovery. (A)** Mean amplitude of compound action potential (CMAP) from ipsilateral gastrocnemius and plantar muscles after sciatic nerve crush of animals treated with vehicle (Veh) or C1 (n=5, ANOVA, post hoc Bonferroni*p<0.05 vs Veh). Right, representative recordings. **(B)** Histogram of the percentage of treated animals that presented electrophysiological evidence of reinenrvation at the plantar muscle at different time-points **(C)** Plot of the sciatic functional index (SFI) obtained with walking track analysis of sciatic nerve crushed animals treated with either vehicle (Veh) or C1. Right, representative footprints from ipsi and contralateral paws, obtained at 35 days post injury (dpi). **(D)** Bar graph showing the percentage of reinnervated motor endplates at plantar muscle with (right) representative pictures of reinnervated neuromuscular junctions showing nerve fibers immunostained for NF200 (red) and end-plates labeled with bungarotoxin (green). **(E)** Microphotographs of spinal MNs at the ventral horn showing no signs of cell death due to nerve crush after 3 weeks post injury.

## DETAILED DESCRIPTION OF THE INVENTION

*Combination*

In the first aspect, the present invention relates to a combination comprising acamprosate or its pharmaceutically acceptable salt thereof, and ribavirin or a pharmaceutically acceptable salt thereof. In the context of the present invention, this combination is also designated as "C1".

Acamprosate (also known as N-acetyl homotaurine) is used to designate the compound 3-acetamidopropane-1-sulfonic acid and has the following chemical structure

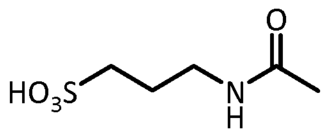
.

Currently, it is marketed under the name Campral® in the form of its calcium salt of formula:

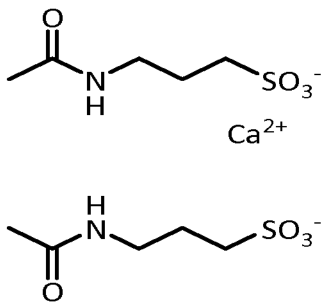

Ribavirin is used to designate the compound 1-[(2R,3R,4S,5R)-3,4-dihydroxy-5-(hydroxymethyl)oxolan-2-yl]-1H-1,2,4-triazole-3-carboxamide and has the following chemical structure

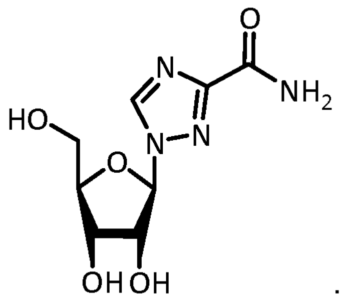
.

The term "combination" or "combination product" refers to a group of 1 or 2 compositions wherein the above mentioned products acamprosate or a pharmaceutically acceptable salt thereof, and ribavirin or a pharmaceutically acceptable salt thereof, are distributed among the 1 or 2 compositions and wherein each of the 1 or 2 compositions comprises at least one of acamprosate or a pharmaceutically acceptable salt thereof, and ribavirin or a pharmaceutically acceptable salt thereof. Examples of combinations are, among others:

- a combination comprising a first composition comprising acamprosate or a pharmaceutically acceptable salt thereof,

and a second composition comprising ribavirin or a pharmaceutically acceptable salt thereof; and

- a combination comprising a composition comprising acamprosate or a pharmaceutically acceptable salt thereof, and ribavirin or a pharmaceutically acceptable salt thereof.

In a preferred embodiment, acamprosate or a pharmaceutically acceptable salt thereof, and ribavirin or a pharmaceutically acceptable salt thereof, form part of the same composition.

In an alternative embodiment, acamprosate or a pharmaceutically acceptable salt thereof, and ribavirin or a pharmaceutically acceptable salt thereof, form part of the different compositions.

The term "pharmaceutically-acceptable salts" embraces salts commonly used to form metal salts and to form addition salts of free acids or free bases. The nature of the salt is not critical, provided that it is pharmaceutically-acceptable. Suitable pharmaceutically-acceptable acid addition salts may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, arylaliphatic, heterocyclic, carboxylic and sulfonic classes of organic acids, example of which are formic, acetic, adipic, butyric, propionic, succinic, glycolic, gluconic, lactic, malic, tartaric, citric, ascorbic, glucuronic, maleic, fumaric, pyruvic, aspartic, glutamic, benzoic, anthranilic, mesylic, 4-hydroxybenzoic, phenylacetic, mandelic, embonic (pamoic), methanesulfonic, ethanesulfonic, ethanedisulfonic, benzenesulfonic, pantothenic, 2-hydroxyethanesulfonic, toluenesulfonic, sulfanilic, cyclohexylaminosulfonic, camphoric, camphorsulfonic, digluconic, cyclopentanepropionic, dodecylsulfonic, glucoheptanoic, glycerophosphonic, heptanoic, hexanoic, 2-hydroxyethanesulfonic, nicotinic, 2-naphthalenesulfonic, oxalic, palmoic, pectinic, persulfuric, 2-phenylpropionic, picric, pivalic propionic, succinic, tartaric, thiocyanic, mesylic, undecanoic, stearic, algenic, [beta]-hydroxybutyric, salicylic, galactaric and galacturonic acid. Suitable pharmaceutically-acceptable base addition salts include metallic salts, such as salts made from aluminum, calcium, lithium, magnesium, potassium, sodium and zinc, or salts made from organic bases including primary, secondary and tertiary amines, substituted amines including cyclic amines, such as caffeine, arginine, diethylamine, N-ethyl piperidine, aistidine, glucamine, isopropylamine, lysine, morpholine, N-ethyl morpholine, piperazine, piperidine, triethylamine, trimethylamine. All of these salts may be prepared by conventional means from the corresponding compound of the invention by reacting, for example, the appropriate acid or base with the compound of the invention. When a basic group and an acid group are present in the same molecule, a compound of the invention may also form internal salts.

In preferred pharmaceutically acceptable salt of acamprosate is the calcium salt of acamprosate (also designated as acamprosate calcium) of formula:

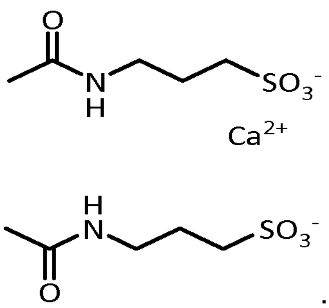

The combination comprising acamprosate or a pharmaceutically acceptable salt thereof, and ribavirin or a pharmaceutically acceptable salt thereof may be formulated using a ratio by weight of acamprosate or a pharmaceutically acceptable salt thereof:ribavirin or a pharmaceutically acceptable salt thereof ranging for example, from 5000:1 to 1:1 by weight of acamprosate or a pharmaceutically acceptable salt thereof:ribavirin or a pharmaceutically acceptable salt thereof, preferably from 500:1 to 1:1, more preferably from 50:1 to 1:1, even more preferably from 10:1 to 1:1, preferably from 5:1 to 1:1, the most preferred from 50:1 to 30:1. In a preferred embodiment, the ratio by weight of acamprosate or a pharmaceutically acceptable salt thereof:ribavirin or a pharmaceutically acceptable salt thereof is from 4100:1 to 40:1. In a particular embodiment the ratio by weight is selected from the group consisting of the following ratios: from 4100:1 to 4000:1, from 410:1 to 400:1, from 190:1 to 170:1, from 41:1 to 40:1 and from 4.1:1 to 4.0:1, preferably, the ratio by weight is from 41:1 to 40:1.

In the context of the present invention, unless otherwise stated, the ratios and amounts of components of the combination described herein refer to the equivalent amount of acamprosate and/or ribavirin in their free form, i.e. without counting the salt forming counterion, even if said compounds are present in the form of a pharmaceutically acceptable salt in the combination. For example, when defining the amount or ratio of acamprosate in a combination comprising said compound as its calcium salt (i.e. acamprosate calcium), said amount or ratio refers to the equivalent amount of acamprosate free acid present in the combination.

*Medical uses of the combination*

The examples of the application show that the combination of the present invention has an unexpected neuroprotective and neuroregenerative effect for motoneurons, reduces inflammatory-related states of the central nervous system after axonal injury and promotes the appearance of pro-regenerative neuronal phenotype.

Thus, the combination of the present invention, which comprises acamprosate or a pharmaceutically acceptable salt thereof, and ribavirin or a pharmaceutically acceptable salt thereof, as described above, is useful for the treatment and/or prevention of neurological damage in disorders selected from the group consisting of lesions of efferent/afferent fibers, lesions of the posterior and ventral roots of the spinal cord or motor axons, and diseases affecting lower motoneurons, as well as other conditions associated with ER stress.

Thus, in one aspect, the present invention relates to a combination comprising acamprosate or a pharmaceutically acceptable salt thereof, and ribavirin or a pharmaceutically acceptable salt thereof, as described above, for use as a medicament.

In a further aspect, the present invention relates to the use of a combination comprising acamprosate or a pharmaceutically acceptable salt thereof, and ribavirin or a pharmaceutically acceptable salt thereof, as described above, for the manufacture of a medicament.

In a further aspect, the present invention relates to a combination comprising acamprosate or a pharmaceutically acceptable salt thereof, and ribavirin or a pharmaceutically acceptable salt thereof, as defined above, for use in the treatment and/or prevention of neurological damage.

As used herein, the term "neurological damage" refers to an injury of the nervous system, in particular to motoneurons. Preferably, "neurological damage" refers to peripheral nerve lesion, and other lesions of efferent and afferent fibers such as polyneuropathies, nerve entrapments and nerve compression syndrome (such as carpal tunnel syndrome, meralgia paresthetica, thoracic outlet syndrome, ulnar nerve entrapment, compressed neuropathy) and nerve traumatism (such as brachial plexus injury, foot drop injury, spinal accessory nerve injury, traumatic nerve injury, complex regional pain syndrome); lesions of the posterior (dorsal) root of the spinal cord like radiculopathies, spinal disc herniations, tumours (such as malignant nerve sheath tumors, neurofibromas, schwannomas) and radiotherapy; lesions or traumatic cranial neuropathy affecting cranial nerves (olfactory, oculomotor, trochlear, abducens, facial, vestibulocochlear, accessory and hypoglossal nerves, trigeminal nerve and ganglion, optic nerve and chiasm); diseases of the motoneurons of the posterior (ventral) root of the spinal cord including acute polyomelitis or motor axons such as Guillain-Barré syndrome; and diseases affecting lower motoneurons like peripheral neuropathies, amyotrophic lateral sclerosis (ALS), spinal muscular atrophy (SMA); acute and chronic inflammatory pain, neuropathic pain, neuritis, phantom limb pain, radiculopathy, traumatic injury. The term "neurological damage" also refers to damage to the nerve caused by neurodegenerative processes ensuing after the implantation of surgical prostheses.

Another aspect of the present invention relates to the use of a combination comprising acamprosate or a pharmaceutically acceptable salt thereof, and ribavirin or a pharmaceutically acceptable salt thereof, as defined above, in the manufacture of a medicament for the treatment and/or prevention of neurological damage, particularly peripheral nerve lesion, and other lesions of efferent and afferent fibers such as polyneuropathies, nerve entrapments and nerve compression syndrome (such as carpal tunnel syndrome, meralgia paresthetica, thoracic outlet syndrome, ulnar nerve entrapment, compressed neuropathy) and nerve traumatism (such as brachial plexus injury, foot drop injury, spinal accessory nerve injury, traumatic nerve injury, complex regional pain syndrome); lesions of the posterior (dorsal) root of the spinal cord like radiculopathies, spinal disc herniations, tumours (such as malignant nerve sheath tumors, neurofibromas, schwannomas) and radiotherapy; lesions or traumatic cranial neuropathy affecting cranial nerves (olfactory, oculomotor, trochlear, abducens, facial, vestibulocochlear, accessory and hypoglossal nerves, trigeminal nerve and ganglion, optic nerve and chiasm); diseases of the motoneurons of the posterior (ventral) root of the spinal cord including acute polyomelitis or motor axons such as Guillain-Barré syndrome; and diseases affecting lower motoneurons like peripheral neuropathies, amyotrophic lateral sclerosis (ALS), spinal muscular atrophy (SMA); acute and chronic inflammatory pain, neuropathic pain, neuritis, phantom limb pain, radiculopathy, traumatic injury. Moreover, the combination is also useful for prevention of inflammatory and neurodegenerative processes ensuing after the implantation of surgical prostheses.

Another aspect relates to a method of treatment and/or prevention of neurological damage, particularly peripheral nerve lesion, and other lesions of efferent and afferent fibers such as polyneuropathies, nerve entrapments and nerve compression syndrome (such as carpal tunnel syndrome, meralgia paresthetica, thoracic outlet syndrome, ulnar nerve entrapment, compressed neuropathy) and nerve traumatism (such as brachial plexus injury, foot drop injury, spinal accessory nerve injury, traumatic nerve injury, complex regional pain syndrome); lesions of the posterior (dorsal) root of the spinal cord like radiculopathies, spinal disc herniations, tumours (such as malignant nerve sheath tumors, neurofibromas, schwannomas) and radiotherapy; lesions or traumatic cranial neuropathy affecting cranial nerves (olfactory, oculomotor, trochlear, abducens, facial, vestibulocochlear, accessory and hypoglossal nerves, trigeminal nerve and ganglion, optic nerve and chiasm); diseases of the motoneurons of the posterior (ventral) root of the spinal cord including

acute polyomelitis or motor axons such as Guillain-Barré syndrome; and diseases affecting lower motoneurons like peripheral neuropathies, amyotrophic lateral sclerosis (ALS), spinal muscular atrophy (SMA); acute and chronic inflammatory pain, neuropathic pain, neuritis, phantom limb pain, radiculopathy, traumatic injury, in a subject in need thereof, which comprises the administration of a therapeutically effective amount of a combination comprising acamprosate or a pharmaceutically acceptable salt thereof, and ribavirin or a pharmaceutically acceptable salt thereof, as defined above. Moreover, the combination is also useful for prevention of inflammatory and neurodegenerative processes ensuing after the implantation of surgical prostheses.

In a particular embodiment of any of the aspects described above, the clinical condition is a traumatic nerve injury, preferably peripheral nerve injury.

In another embodiment, the present combinations may also be used with other types of therapies for treating and/or preventing other lesions of efferent and afferent fibers such as polyneuropathies, nerve entrapments and nerve compression syndrome (such as carpal tunnel syndrome, meralgia paresthetica, thoracic outlet syndrome, ulnar nerve entrapment, compressed neuropathy) and nerve traumatism (such as brachial plexus injury, foot drop injury, spinal accessory nerve injury, traumatic nerve injury, complex regional pain syndrome); lesions of the posterior (dorsal) root of the spinal cord like radiculopathies, spinal disc herniations, tumours (such as malignant nerve sheath tumors, neurofibromas, schwannomas) and radiotherapy; lesions or traumatic cranial neuropathy affecting cranial nerves (olfactory, oculomotor, trochlear, abducens, facial, vestibulocochlear, accessory and hypoglossal nerves, trigeminal nerve and ganglion, optic nerve and chiasm); diseases of the motoneurons of the posterior (ventral) root of the spinal cord including acute polyomelitis or motor axons such as Guillain-Barré syndrome; and diseases affecting lower motoneurons like peripheral neuropathies, amyotrophic lateral sclerosis (ALS), spinal muscular atrophy (SMA); acute and chronic inflammatory pain, neuropathic pain, neuritis, phantom limb pain, radiculopathy, traumatic injury and for the prevention of inflammatory and neurodegenerative processes ensuing after the implantation of surgical prostheses.

In another embodiment, the present combinations may also be used or administered in combination with other drugs for the treatment and/or prevention of traumatic nerve injury, preferably peripheral nerve injury.

As used herein, the terms "treat", "treatment" and "treating" refer to the reduction or amelioration of the progression, severity and/or duration of a disease or disorder, or the amelioration of one or more symptoms (preferably, one or more discernible symptoms) related a disease or disorder.

As used herein, the terms "prevent", "prevention" and "preventing" refer to the reduction in the risk of acquiring or developing a given disease or disorder, or the reduction or inhibition of the recurrence or a disease or disorder.

As used herein, the terms "subject", "patient" are used interchangeably. The terms "subject" and "patient" refer to an animal (e.g., a bird such as a chicken, quail or turkey, or a mammal), preferably a mammal including a non-primate (e.g., a cow, pig, horse, sheep, rabbit, guinea pig, rat, cat, dog, and mouse) and a primate (e.g., a monkey, chimpanzee and a human), and more preferably a human. In one embodiment, the subject is a non-human animal such as a farm animal (e.g., a horse, cow, pig or sheep), or a pet (e.g., a dog, cat, guinea pig or rabbit). In another embodiment, the subject is a human.

As used herein, the term "therapeutically effective amount" refers to an amount of a combination of this invention which is sufficient to reduce or ameliorate the severity, duration, progression, or onset of neurological damage, particularly peripheral nerve lesion, and other lesions of efferent and afferent fibers such as polyneuropathies, nerve entrapments and nerve compression syndrome (such as carpal tunnel syndrome, meralgia paresthetica, thoracic outlet syndrome, ulnar nerve entrapment, compressed neuropathy) and nerve traumatism (such as brachial plexus injury, foot drop injury, spinal accessory nerve injury, traumatic nerve injury, complex regional pain syndrome); lesions of the posterior (dorsal) root of the spinal cord like radiculopathies, spinal disc herniations, tumours (such as malignant nerve sheath tumors, neurofibromas, schwannomas) and radiotherapy; lesions or traumatic cranial neuropathy affecting cranial nerves (olfactory, oculomotor, trochlear, abducens, facial, vestibulocochlear, accessory and hypoglossal nerves, trigeminal nerve and ganglion, optic nerve and chiasm); diseases of the motoneurons of the posterior (ventral) root of the spinal cord including acute polyomelitis or motor axons such as Guillain-Barré syndrome; and diseases affecting lower motoneurons like peripheral neuropathies, amyotrophic lateral sclerosis (ALS), spinal muscular atrophy (SMA); acute and chronic inflammatory pain, neuropathic pain, neuritis, phantom limb pain, radiculopathy, traumatic injury and for the prevention of inflammatory and neurodegenerative processes ensuing after the implantation of surgical prostheses, at a reasonable benefit/risk ratio applicable to any medical treatment, or prevent the advancement of a the diseases described above, cause the regression of the diseases described above, prevent the recurrence, development, onset or progression of a symptom associated with the diseases described above, or enhance or improve the prophylactic or therapeutic effect(s) of another therapy. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

In a particular embodiment the combination of the present invention comprising acamprosate or a pharmaceutically acceptable salt thereof, and ribavirin or a pharmaceutically acceptable salt thereof is used in a dosage from 0.0002 mg to 152500 mg of acamprosate or a pharmaceutically acceptable salt thereof per $m^2$ of body weight per day, preferably from 0.0002 mg/$m^2$/day to 8000 mg/$m^2$/day of acamprosate or a pharmaceutically acceptable salt thereof, and a dosage of from 0.0000003 mg/$m^2$/day to 110400 mg/$m^2$/day of ribavirin or a pharmaceutically acceptable salt thereof, preferably from 0.0000003 mg/$m^2$/day to 6000 mg/$m^2$/day of ribavirin or a pharmaceutically acceptable salt thereof wherein the amount of compound is expressed with respect to the free form of the drug, i.e. either as free acamprosate or as free ribavirin.

Preferably, the combination of the present invention comprising acamprosate or a pharmaceutically acceptable salt thereof, and ribavirin or a pharmaceutically acceptable salt thereof is used in a dosage from 0.014 mg/$m^2$/day to 1500 mg/$m^2$/day of acamprosate or a pharmaceutically acceptable salt thereof, and a dosage of from 0.000349 mg/$m^2$/day to 1100 mg/$m^2$/day of ribavirin or a pharmaceutically acceptable salt thereof, in particular for parenteral administration; preferably from 0.014 mg/$m^2$/day to 1.5 mg/$m^2$/day of acamprosate or a pharmaceutically acceptable salt thereof, and a dosage of from 0.000349 mg/$m^2$/day to 0.0349 mg/$m^2$/day of ribavirin or a pharmaceutically acceptable salt thereof, for intrathecal administration, such as to the cerebrospinal fluid; preferably from 15 mg/$m^2$/day to 1500 mg/$m^2$/day of acamprosate or a pharmaceutically acceptable salt thereof, and a dosage of from 11 mg/$m^2$/day to 1100 mg/$m^2$/day of ribavirin or a pharmaceutically acceptable salt thereof for intravenous, intramuscular, intradermic or hypodermic administration. Preferably, the combination of the present invention comprising acamprosate or a pharmaceutically acceptable salt thereof, and ribavirin or a pharmaceutically acceptable salt thereof is used in a dosage from 76 mg/$m^2$/day to 7600 mg/$m^2$/day of acamprosate or a pharmaceutically acceptable salt thereof, and a dosage of from 55.2 mg/$m^2$/day to 5520 mg/$m^2$/day of ribavirin or a pharmaceutically acceptable salt thereof, in particular for oral administration. Preferably, the combination of the present invention comprising acamprosate or a pharmaceutically acceptable salt thereof, and ribavirin or a pharmaceutically acceptable salt thereof is used in a dosage from 3.5 mg/$m^2$/day to 152500 mg/$m^2$/day of acamprosate or a pharmaceutically acceptable salt thereof, and a dosage of from 2 mg/$m^2$/day to 110400 mg/$m^2$/day of ribavirin or a pharmaceutically acceptable salt thereof, in particular for topical administration.

Doses of active ingredients may be expressed either in mg of active ingredient per kg of body weight or in mg of active ingredient per square meter of body surface. The article from Reagan-Shaw S. "Dose translation from animal to human studies revisited". FASEB J 2007, 22:659-661 provides the standard conversion factors used to convert mg/kg to mg/$m^2$.

$$\text{Dose (mg/kg)} \times K_m = \text{Dose (mg/m}^2\text{)}$$

The article also explains that this conversion is the basis for converting dose in a first animal species to dose in a second animal species (allometric dose translation). Thus, animal dose (AD) in mg/kg can be converted to human equivalent dose (HED) in mg/kg using the following formula:

$$\text{HED (mg/kg)} = \text{AD (mg/kg)} \times \frac{\text{Animal } K_m}{\text{Human } K_m}$$

wherein the $K_m$ for each species is shown in Table 2 (data extracted from Reagan-Shaw S. "Dose translation from animal to human studies revisited". FASEB J 2007, 22:659-661).

Table 2. $K_m$ factor for conversion of AD to HED

| *Species* | | *$K_m$ factor* |
|---|---|---|
| Human | Adult | 37 |
| | Child | 25 |
| Baboon | | 20 |
| Dog | | 20 |
| Monkey | | 12 |

(continued)

| *Species* | $K_m$ *factor* |
|---|---|
| Rabbit | 12 |
| Guinea pig | 8 |
| Rat | 6 |
| Hamster | 5 |
| Mouse | 3 |

Acamprosate or a pharmaceutically acceptable salt thereof and ribavirin or a pharmaceutically acceptable salt thereof, comprised in the combination of the present invention, may be administered simultaneously, sequentially or separately. In any of these particular administration modes, the combination according to the present invention is designed to enable the simultaneous, sequential or separate administration of acamprosate or a pharmaceutically acceptable salt thereof and ribavirin or a pharmaceutically acceptable salt thereof.

Simultaneous administration may, e.g., take place in the form of one composition comprising acamprosate or a pharmaceutically acceptable salt thereof and ribavirin or a pharmaceutically acceptable salt thereof, or by simultaneously administering, i.e. administering at the same time, acamprosate or a pharmaceutically acceptable salt thereof, and acamprosate or a pharmaceutically acceptable salt thereof which are formulated independently, i.e. not forming part of the same composition.

Sequential administration preferably means administration of acamprosate or a pharmaceutically acceptable salt thereof, at one time point, and ribavirin or a pharmaceutically acceptable salt thereof at a different time point, in a chronically staggered manner.

Separate administration preferably means administration of acamprosate or a pharmaceutically acceptable salt thereof, and ribavirin or a pharmaceutically acceptable salt thereof at a different time point, independently of each other at different time points.

When administered sequentially or separately, acamprosate or a pharmaceutically acceptable salt thereof, and ribavirin or a pharmaceutically acceptable salt thereof, may be administered in any order. In one particular embodiment, acamprosate or a pharmaceutically acceptable salt thereof, is administered first. In another particular embodiment, ribavirin or a pharmaceutically acceptable salt thereof is administered first. The particular period of delay between the administration of the individual compounds in the combination can extend to days, hours, minutes or seconds.

In a particular embodiment the combination of the present invention comprises acamprosate or a pharmaceutically acceptable salt thereof, and ribavirin or a pharmaceutically acceptable salt thereof, as defined above, forming part of the same composition for simultaneous administration.

In another particular embodiment, the combination according to the present invention comprises acamprosate or a pharmaceutically acceptable salt thereof, and ribavirin or a pharmaceutically acceptable salt thereof, as defined above, provided as separate compositions, preferably for simultaneous administration.

These separate compositions are also suitable for sequential and separate administration.

*Pharmaceutical Compositions*

This invention also provides pharmaceutical compositions that comprise the combination of this invention wherein the acamprosate or a pharmaceutically acceptable salt thereof and ribavirin or a pharmaceutically acceptable salt thereof, are formulated together with one or more pharmaceutically acceptable carriers in a single composition. The pharmaceutical compositions may be specially formulated for any mode of administration that is medically acceptable, meaning any mode that produces effective levels of the active compounds without causing clinically unacceptable adverse effects.

In one preferred embodiment, compositions of the invention are administered orally. Other modes of administration include topical, buccal, transdermal, within/on implants, or parenteral routes. The term "parenteral" includes subcutaneous, intrathecal, intraventricular, intravenous, nasal, intraocular, intramuscular, intraperitoneal, intraarticular, subarachnoid, *dorsalis* venous plexis injection or infusion or direct infusion to the cerebrospinal fluid. The pharmaceutical compositions of the invention may be in the form of conjugates, gels, liposomes, microspheres, nanoparticles, carriers, pumps, catheters, implants, controlled-release microchips, and the similar. Pharmaceutical compositions of the invention can be added to a physiological fluid.

The term "pharmaceutically acceptable carrier" as used herein means a non-toxic, inert solid, semi-solid or liquid filler, diluent, encapsulating material or formulation auxiliary of any type. Some examples of materials which can

serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. This invention provides pharmaceutical compositions which comprise compounds of the invention formulated together with one or more non-toxic pharmaceutically acceptable carriers. Pharmaceutical compositions of this invention for injection comprise pharmaceutically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethylene glycol, glycerol, 1,3-butanediol and the like), suitable mixtures thereof, vegetable oils (such as olive oil) and injectable organic esters such as ethyl oleate, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. Proper fluidity may be maintained, for example, by the use of a coating such as lecithin, by the maintenance of the required particle size in the case of dispersions, and by the use of surfactants.

These pharmaceutical compositions may also contain adjuvants such as preservative agents, wetting agents, emulsifying agents, and dispersing agents. Prevention of the action of microorganisms may be ensured by various antibacterial and antifungal agents, for example, parabens, chlorobutanol, phenol, sorbic acid, and the like. It may also be desirable to include isotonic agents, for example, sugars, sodium chloride and the like. Prolonged absorption of the injectable pharmaceutical form may be brought about by the use of agents delaying absorption, for example, aluminum monostearate and gelatin.

In some cases, in order to prolong the effect of a drug, it is often desirable to slow the absorption of the drugs (acamprosate or a pharmaceutically acceptable salt thereof, and ribavirin or a pharmaceutically acceptable salt thereof) from subcutaneous or intramuscular injection. This may be accomplished by the use of a liquid suspension of crystalline or amorphous material with poor water solubility. The rate of absorption of the drugs then depends upon its rate of dissolution which, in turn, may depend upon crystal size and crystalline form. Alternatively, delayed absorption of a parenterally administered drugs is accomplished by dissolving or suspending the drugs in an oil vehicle.

Suspensions, in addition to the active compounds (acamprosate or a pharmaceutically acceptable salt thereof, and ribavirin or a pharmaceutically acceptable salt thereof), may contain suspending agents, as, for example, ethoxylated isostearyl alcohols, polyoxyethylene sorbitol and sorbitan esters, microcrystalline cellulose, aluminum metahydroxide, bentonite, agar-agar, tragacanth, and mixtures thereof.

If desired, and for more effective distribution, acamprosate or a pharmaceutically acceptable salt thereof, and ribavirin or a pharmaceutically acceptable salt thereof comprised in the combination of this invention can be incorporated into slow-release or targeted-delivery systems such as polymer matrices, liposomes, and microspheres.

The pharmaceutical compositions of the invention may be sterilized, for example, by filtration through a bacteria-retaining filter or by incorporation of sterilizing agents in the form of sterile solid compositions, which may be dissolved in sterile water or some other sterile injectable medium immediately before use.

The active compounds (acamprosate or a pharmaceutically acceptable salt thereof, and ribavirin or a pharmaceutically acceptable salt thereof) can also be in micro-encapsulated form, if appropriate, with one or more pharmaceutically acceptable carriers as noted above. The solid dosage forms of tablets, dragees, capsules, pills, granules and device can be prepared with coatings and shells such as enteric coatings, release controlling coatings and other coatings well known in the pharmaceutical formulating art. In such solid dosage forms the active compound can be admixed with at least one inert diluent such as sucrose, lactose, or starch. Such dosage forms may also comprise, as is normal practice, additional substances other than inert diluents, e.g., tableting lubricants and other tableting aids such as magnesium stearate and microcrystalline cellulose. In the case of capsules, tablets and pills, the dosage forms may also comprise buffering agents. They may optionally contain opacifying agents and can also be of such composition that they release the active ingredient(s) only, or preferentially, in a certain part of the intestinal tract in a delayed manner. Examples of embedding compositions which can be used include polymeric substances and waxes.

Injectable depot forms are made by forming microencapsulated matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

Solid dosage forms for oral administration include capsules, tablets, pills, powders, and granules. In such solid dosage forms, the active compound is mixed with at least one inert pharmaceutically acceptable carrier such as sodium citrate or calcium phosphate and/or a) fillers or extenders such as starches, lactose, sucrose, glucose, mannitol, and salicylic acid; b) binders such as carboxymethylcellulose, alginates, gelatin, polyvinylpyrrolidinone, sucrose, and acacia; c) humectants such as glycerol; d) disintegrating agents such as agar-agar, calcium carbonate, potato or tapioca starch, alginic acid, certain silicates, and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay; and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract in a delayed manner. Examples of embedding compositions which can be used include polymeric substances and waxes.

Compositions for rectal administration are preferably suppositories which can be prepared by mixing the compounds of this invention with suitable non-irritating carriers such as cocoa butter, polyethylene glycol or a suppository wax which are solid at ambient temperature but liquid at body temperature and therefore melt in the rectum and release the active compound.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Besides inert diluents, the oral compositions can also include adjuvants such as wetting agents, emulsifying and suspending agents, sweetening, flavoring, and perfuming agents.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, lactose, talc, silicic acid, aluminum hydroxide, calcium silicates and polyamide powder, or mixtures of these substances. Sprays can additionally contain customary propellants such as chlorofluorohydrocarbons.

Compounds of this invention may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes may be used. The present compositions in liposome form may contain, in addition to the compounds of this invention, stabilizers, preservatives, and the like. The preferred lipids are the natural and synthetic phospholipids and phosphatidylcholines (lecithins) used separately or together.

Methods to form liposomes are known in the art. See, for example, Prescott, Ed., Methods in Cell Biology, Volume XIV, Academic Press, New York, N.Y., (1976), p 33 et seq.

The pharmaceutical compositions of the invention are suitable for the same medical uses and methods of treatment and/or prevention as defined above for the combinations of the present invention.

*Kits containing compositions*

The invention also relates to a kit, wherein the individual compounds of the combination, i.e. Acamprosate or a pharmaceutically acceptable salt thereof, and Ribavirin or a pharmaceutically acceptable salt thereof are disposed in

separate containers.

The invention also relates to a kit where, additionally to the compounds of the combination, other devices such as injection devices, and other materials such as pharmaceutical carriers, are included.

The invention also relates to a kit according to any of the foregoing, further comprising integrally thereto or as one or more separate documents, information pertaining to the contents or the kit and the use thereof.

The term "comprising" is meant to be open ended, including the indicated component but not excluding other elements.

Administration may include a single daily dose or administration of a number of discrete divided doses as may be appropriate. An administration regime may also include administration of one or more of the active agents, or compositions comprising same, as described herein. The period of administration may be variable. It may occur for as long a period is desired.

The following examples further illustrate specific embodiments of the invention; however, the following illustrative examples should not be interpreted in any way to limit the extent of the invention.

EXAMPLES

EXAMPLE 1

**Experimental model**

The efficacy of a selected drug combination C1, comprising Acamprosate calcium and Ribavirin, to face motoneuron degeneration was studied *in silico, in vivo* and *in vitro.*

*In silico,* we performed a study of the mode of action (MoA).

Characterization of root avulsion and distal axotomy. We obtained an initial list of key proteins associated to molecular processes or motives that could be involved in the responses triggered after neuronal/axonal damage extracted by literature scrutiny. We selected those with high content on pathways and protein complexes linked to either neuroprotection/regeneration or degeneration/pain. The human biological network incorporates all the available relationships between proteins from a regularly updated in-house database drawn from public sources: KEGG35, REACTOME36, INTACT (Brooks bank et al., 2003), BIOGRID38, MINT (PMID: 22096227). The analysis was focused in the area around regeneration and degeneration of the human biological network, which included 3,296 proteins for Regeneration; 3,836 proteins for Degeneration, and 1,267 proteins for Neuropathic Pain. We found that several proteins were overlapped 856 between degeneration/pain, 2,232 between degeneration/regeneration, 858 between regeneration/pain.

Construction of DA (Distal Axotomy) and RA (Root Avulsion) mathematical models. Sampling Methods are used to generate RA and DA mathematical models that comply with a experimental proteomic data available and the set of restrictions, corresponding to the available biological knowledge about the constructed networks, together with knowledge derived from DrugBank, GEO (PMCID: PMC3531084) and MALDI-Toff Mass spectrometry data generated from RA and DA rat model. Each plausible sample model is constructed using stochastic optimization algorithms. These algorithms use probabilistic measures derived from the biological evidences to adjust network interaction types and strengths. A model comparison analysis is used to identify the key proteins that trigger degeneration into regeneration.

Repositioning analysis. The list of key proteins identified as key for the reversion of degenerative model to regenerative model are used as starting point for the repositioning analysis. Two complementary strategies were used to generate mathematical models of living systems: an Artificial Neural Network (ANNs) and Sampling Methods. ANNs are supervised algorithms which identify relations between drug targets and clinical elements of the network that are used for training a classifier with the information contained in DrugBank about drugs and indications (PubMed ID: 18048412; PubMed ID: 16381955), with capacity for predicting and scoring novel potential drugs. The accuracy of the ANNs to reproduce the indications of Drugbank is 98% for those drugs with all targets in the human biological network. A total of 5440 drugs that generated approximately 15 million of binary combinations were screened. Combinations were sorted and selected by its relationship with RA, outstanding safety profile that did not lead to neuropathic pain, high synergism and no previous relationship with CNS/PNS regeneration. Sampling methods were also used to describe with high capability all plausible relationship between sets of proteins previously identified with ANNs. Sampling Methods generate mathematical models that comply with a given set of restrictions described above. As the number of restrictions is always smaller than the number of parameters required by the algorithm, any process modelled by TPMS has a "population" of different solutions, which is set around 106-109, since this interval is estimated to faithfully portray nature. Consequently, models result in both "global" predicted MoA, which account for the majority of the population, and "cluster" mechanisms of action, which are more accurate for population subgroups. A normalized synergism score (SE) based on the values of the ANN prediction and the analysis of the MoA obtained from the Sampling Methods is provided for the protein involved in the synergism between the two drugs of the combination. SE of a protein is computed simulating the pattern of activation of the protein for different drugs doses and drug combination mixture ratios. These patterns are

used to classify the protein behaviour as independent to drugs, dependent of one drug, affected additively, or synergistically (sub additive or super additive). Finally SE accounts for the weighted average of the percentage of model solutions that present the node of interest being affected by both drugs, either in an additive, synergistic or antagonistic way, and the synergistic effect over the node of both drugs (rather than additive).

The MoA is validated in a two-step process. First, we checked that each relation of proteins involved in the MoA between each other can be contrasted with the literature. Second, we checked that the MoA made sense overall, featuring pathways coherent with the living system, the combinations of repositioned drugs used for treatment and the known pathophysiology of RA.

For *in vivo* studies, a pre-clinical rat model of peripheral nerve root avulsion (RA model) was generated as previously described (see Penas C Cytoskeletal and activity-related changes in spinal motoneurons after root avulsion. Journal of Neurotrauma, 2009). Briefly, a midline skin incision was made at the level of the iliac crest, the right sciatic nerve was identified and the L4 and L5 spinal nerves were separated. A moderate traction was applied to produce extravertebral nerve root avulsion, leading to a retrograde degeneration of around 80% of the injured MNs within a month. The left roots were kept intact as control sham. We intratechally administered the vehicle (artificial cerebrospinal fluid (aCSF) [NaCl (124 mM), KCl (3 mM), $NaHCO_3$ (26 mM), $CaCl_2/2H_2O$ (2 mM), $MgSO_4/7H_2O$ (1 mM), $KH_2PO_4$ (1.25 mM) and D-Glucose (10 mM)] or the positive control drug for neuroprotection (PRE084, 1-phenylcyclohexanecarboxylate hydrochloride, 1.7 μg/kg/day), C1 (Acamprosate calcium 96 μg/Kg/day (equivalent to 86.9 μg/Kg/day acamprosate free acid) + Ribavirin 0.48 μg/Kg/day) or acamprosate calcium (96 μg/Kg/day) (equivalent to 86.9 μg/Kg/day acamprosate free acid) alone and ribavirin (0.48 μg/Kg/day) alone. For this purpose, we used a programmable micro-infusion pump system (iPrecio, Primetech) to continuously perfuse the drugs into the animal CSF during 20 days from the following day after RA. Seven experimental animal groups with RA were defined: one was treated with C1, another was treated with Pre084 (positive control), another was treated with Acamprosate calcium alone (A), another was treated with Ribavirin alone (B), another was treated with vehicle alone, another was left untreated (control). Pre084 is a sigma receptor selective for the σ1 subtype which is known to promote neuroprotection (Penas C, Sigma receptor agonist 2-(4-morpholinethyl)1 phenylcyclohexanecarboxylate (Pre084) increases GDNF and BiP expression and promotes neuroprotection after root avulsion injury, J Neurotrauma. 2011). After 3 weeks, animals were perfused and L4-L5 segments of the spinal cord were removed, post-fixed and cryopreserved until sectioning with a crysotate. We stained spinal cord sections with fluorescent Nissl (green) to reveal alpha MNs at the Rexed's lamina IX of the ventral horn, recognized by its localization, big size and prominent nuclei. Motoneuron (MN) counts were performed by comparison between the number of ventral horn MNs in the ipsilateral side and the contralateral side of the same spinal cord section/animal. For immunohistochemical studies, the slices containing the spinal cord sections were basically blocked using 10% of different species sera in standard Tris-buffered saline (TBS) including 0.3% triton, and then incubated with primary antibodies of several markers including anti-GFAP, anti-ibal and anti-GAP43 overnight. After several washes and incubation with adequate fluorescent secondary antibodies, the slices were mounted with fluoromount to be prepared for microscopical analysis. Images from microscope were digitally captured and analyzed using several plugins of the ImageJ Software.

For the *in vitro* studies, we assessed cell survival of the NSC-34 motoneuron-like cells with a conventional MTT-based assay. NSC-34 is a hybrid cell line produced by fusion of neuroblastoma with mouse motoneuron-enriched primary spinal cord cells, which express properties characteristic of MNs, including the generation of action potentials, the presence of specific neurofilaments and acetylcholine signaling. In order to mimic ER stress, an alteration found to contribute to neurodegeneration after root avulsion (see Penas C, Autophagy, and BiP level decrease are early key events in retrograde degeneration of motoneurons, Cell Death Differ. 2011), we used different concentrations of tunicamycin. Tunicamycin is a bacterial toxin which inhibits N-linked glycosylation of newly synthesized proteins, resulting in the activation of the stress-related pathways, and hence is widely used as a classical ER stress inducer. Tunicamycin is a power that was dissolved in DMSO to 10 mg/ml final concentration and then diluted in Dulbeco's modified eagle medium (DMEM) medium at the final working solution indicated in figure legends. The rest of drugs, C1, Acamprosate calcium and Ribavirin were dissolved in water and then in DMEM medium at the final working solutions: 0.11 mM Acamprosate calcium (ACA), 4 μM Ribavirin (RIB) alone or in C1, at a final volume of 100 μl for MTT assays.

### Results on C1 effect on neuroprotection, anti-inflammation and regeneration *in vivo*

As shown in Figure 1, C1 treatment of root avulsed animals significantly increased MN survival compared to the vehicle group. Besides, C1 treatment is not significantly different from the treatment with our positive control (Pre084) which was also significantly different to the vehicle group.

Analysis of glial immunoreactivity after the treatments revealed that C1 significantly reduced the inflammatory state at the central nervous system produced by RA, reducing on both astrogliosis (GFAP positive) and microgliosis (Iba1 positive) (Figure 2, *top and middle panels respectively*). Analysis of the pro-regenerative marker GAP43 in the axonal out branches at the lateral-ventral side of the injured spinal cord segments, revealed levels raised after the treatment with C1 (Figure 2, *bottom*).

**Results on supra-addictive effect of C1 for neuroprotection *in vitro***

We assessed the neuroprotective effect of C1 (acamprosate calcium 0.11 mM + ribavirin 4 μM in 100 μl final volume) when added simultaneous to tunicamycin (TN) in the culture medium. We evaluated cell survival 24 h after the addition of toxin and therapeutic drugs performing an MTT conventional test. As displayed in the figure 3B, C1 significantly protected cells against this stress (77.1%), p<0.05). Note that Pre084 did not neuroprotect the cells as efficiently as C1. The values represented in Figure 3B are gathered in Table 3.

Table 3. MTT assay (% sample vs control)

| | Mean | SEM (±) |
|---|---|---|
| Control | 100 | 0 |
| Vehicle | 96.08 | 1.57 |
| TN 0.1 μg/mL | 82.03 | 3.47 |
| TN 1 μg/mL | 59.37 | 2.34 |
| TN 10 μg/mL | 60.21 | 5.40 |
| PRE084 + TN 1 μg/mL | 68.81 | 4.74 |
| C1 + TN 1 μg/mL | 77.09 | 2.29 |
| Aca+ TN 1 μg/mL | 65.80 | 3.74 |
| Rib+ TN 1 μg/mL | 68.07 | 1.74 |

In order to analyze the existence of possible additive neuroprotective effect between drugs, we determined the dose-effect relations of each individual drug (Acamprosate calcium 0.11 mM or Ribavirin 4 μM, both in 100 μl final volume) compared to equivalent doses for C1 (acamprosate calcium 0.11 mM + ribavirin 4 μM in 100 μl final volume). The results are shown in Tables 4-6.

Table 4: Administration of acamprosate calcium alone. The response is the % of neuronal cell survival after ER stress induced by tunicamycin and measured using an MTT assay. DA is 0.11 mM acamplosate calcium (which corresponds to a Dose DA=1, i.e. 1X) and the corresponding dilutions, i.e. Doses DA from 0 to 1X, being 1X 0.11 mM acamprosate calcium. Corrected Responses are the Responses for each Doses of DA less the control experiment (DA=0, Response=62.956%). N is the number of replicas.

| Doses DA (X) | Response (%) | Corrected Response | N |
|---|---|---|---|
| 0 | 62.956 | 0.00 | 19 |
| 0.1 | 66.475 | 3.52 | 3 |
| 0.2 | 65.918 | 2.96 | 3 |
| 0.5 | 66.849 | 3.89 | 3 |
| 1 | 65.417 | 2.46 | 9 |

Table 5: Administration of ribavirin alone. The Response is the % of neuronal cell survival after ER stress induced by tunicamycin and measured using an MTT assay. DB is 4 μM ribavirin (which corresponds to a Dose DB=1, i.e. 1X) and the corresponding dilutions, Doses DB from 0 to 1X, being 1X 4 μM ribavirin. Corrected Responses are the Responses for each Doses of DB less the control experiment (DB=0, Response=62.956%). N is the number of replicas.

| Doses DB (X) | Response (%) | Corrected Response | N |
|---|---|---|---|
| 0 | 62.956 | 0.00 | 19 |
| 0.1 | 68.662 | 5.71 | 3 |
| 0.2 | 70.886 | 7.93 | 3 |
| 0.5 | 65.676 | 2.72 | 3 |

(continued)

| Doses DB (X) | Response (%) | Corrected Response | N |
|---|---|---|---|
| 1 | 68.647 | 5.69 | 9 |

Table 6: Administration of C1 (combination comprising acomprosate calcium + ribavirin). The Response is the % of neuronal cell survival after ER stress induced by tunicamycin and measured using an MTT assay. DA is 0.11 mM (which corresponds to a Dose DA=1, i.e. 1X), DB is 4 μM (which corresponds to a Dose DB=1, i.e. 1X) and the corresponding dilutions of them. Corrected Responses are the Responses for each Doses of DA less the control experiment (DA=0 or DB=0, Response=62.956%). N is the number of replicas.

| Doses DA (X) | Doses DB (X) | Response (%) | Corrected Response | N |
|---|---|---|---|---|
| 0 | 0 | 62.956 | 0.00 | 19 |
| 0.1 | 0.1 | 76.676 | 13.72 | 3 |
| 0.1 | 1 | 80.095 | 17.14 | 3 |
| 0.5 | 0.5 | 74.301 | 11.35 | 3 |
| 1 | 0.1 | 80.595 | 17.64 | 3 |
| 1 | 1 | 77.094 | 14.14 | 7 |

The analysis of the synergy is summarized in Table 7, wherein the Response is the % of neuronal cell survival after ER stress induced by tunicamycin using an MTT assay. DA is acamprosate calcium 0.11 mM (1X), DB is ribavirin 4 μM (1X) and the corresponding dilutions of them. Obtained Responses of DA, DB (administration of the drugs alone) and C1 (administration of the drugs in combination) are the corrected values from Tables 3-5. The Expected Responses DA+DB is the sum of Obtained Responses of DA when administered alone and DB when administered alone. Difference Obtained vs Expected is the substation between Expected Response and the Obtained response of DA+DB. Ratio of response in front of expected is the ratio of the Difference Obtained vs Expected over the Expected Response of C1 expressed in percentage.

Table 7

| Doses DA | Doses DB | Obtained Response C1 | Obtained Response DA | Obtained Response DB | Expected Response DA+DB | Difference Obtained vs Expected | Ratio of response in front expected |
|---|---|---|---|---|---|---|---|
| 0 | 0 | 0.00 | 0.00 | 0.00 | 0.00 | 0.00 | 0.00 |
| 0.1 | 0.1 | 13.72 | 3.52 | 5.71 | 9.23 | 4.49 | 49% |
| 0.1 | 1 | 17.14 | 3.52 | 5.69 | 9.21 | 7.93 | 86% |
| 0.5 | 0.5 | 11.35 | 3.89 | 2.72 | 6.61 | 4.74 | 72% |
| 1 | 0.1 | 17.64 | 2.46 | 5.71 | 8.17 | 9.47 | 116% |
| 1 | 1 | 14.14 | 2.46 | 5.69 | 8.15 | 5.99 | 73% |

In the assayed doses, the average of the synergy of all experiments is 79% of synergy measured as the ration of the response obtained in front of the expected.

**Results to determine C1 Mechanism of Action**

Experiments in silico based on system biology approach, allowed us to hypothesize the mechanism of action for C1 to neuroprotect. In table 8 we list some of key molecules of this mechanism. Note that at least three of them (XIAP, AIFM1 and PARP1) are directed related to apoptosis. In order to validate some of these relevant molecules (B1 subunit of integrin (itgb1), dynactin (DCTN1) and sirtuin1 (SIRT1)), or related molecules kinesin 5c (kif5c), we performed immunohistochemical analysis.

Table 8. Candidate key proteins to mechanism of action in C1 synergism

| *Uniprot ID* | *Name* | *Effect* |
|---|---|---|
| P08592 | A4 | Amyloid beta A4 protein |
| P28023 | DCTN1 | Dynactin subunit 1 |
| Q9R0I6 | XIAP | E3 ubiquitin-protein ligase XIAP |
| Q9JM53 | AIFM1 | Apoptosis-inducing factor 1 |
| P11980 | KPYM | Pyruvate kinase PKM |
| F1LTP2 | SIRT1 | Protein Sirt1 |
| D4A2G9 | RANG | Protein RGD1565297 |
| Q04589 | FGFR1 | Fibroblast growth factor receptor 1 (FGFR-1) |
| P26051 | CD44 | CD44 antigen |
| P27008 | PARP1 | Poly [ADP-ribose] polymerase 1 |

As shown in figure 4, the treatment with C1 modulated the level of expression of these proteins as expected.

Considering that SIRT1 has been reported to be an important key molecule for neuroprotection and life-span, we focused in determining its relevance in C1 neuroprotection. In order to ascertain whether C1 treatment promote SIRT1 activity, we treated RA-injured animals with C1 concomitantly to the use of either an SIRT1 specific inhibitor of its activity, EX-527 (6-chloro-2,3,4,9-tetrahydro-1H-carbazole-1-carboxamide), or with spermidine. Spermidine acts reducing the levels of acetylated proteins, thus mimicking some effects of SIRT1 increase activity. As shown in figure 5, the use of EX-527 in combination with C1, abolished the neuroprotective effect exerted by C1 while spermidine did maintain it.

**Functional recovery by C1 treatment**

Verification of the regenerative potential of C1 using a model of crush injury to the sciatic nerve. To assess functional recovery of denervated muscles, the compound muscle action potentials (CMAP) evoked in response to sciatic nerve stimulation were recorded in gastronecmius and plantar muscles. We observed that C1 treatment led to a significant increase in CMAP amplitude in both muscles during the follow-up **(Figure 6A).** After 28 days, all treated animals showed evidence of reinnervation of the plantar muscle, compared to 40% of the untreated animals (**Figure 6B**). Furthermore, the recovery of motor function assessed by the sciatic functional index (SFI) revealed a significant improvement in C1-treated animals (**Figure 6C**). Accordingly, quantification of the number of re-innervated neuromuscular junctions (NMJs), assessed by colocalization of NF-200 with alpha-bungarotoxin, showed increased number in C1 treated animals as compared to vehicle-treated ones (**Figure 6D).** As expected, we verified that the number of motoneurons (MNs) remained unaltered in the crush model (**Figure 6E**).

*Electrophysiological and functional examination*

All experiments were conducted in a blinded fashion. For electrophysiological examination, rats were anaesthetized with a cocktail of ketamine/xylazine 0.1 mU100 g weight i.p. The sciatic nerve was stimulated by transcutaneuous electrodes placed at the sciatic notch and the compound muscle potential (CMAP) recorded by placing electrodes on the gastrocnemius and the plantar interosseus muscles. The evoked action potentials were displayed on a storage oscilloscope (Synergy Medelec, Viasys HealthCare) at settings appropriate to measure the amplitude from baseline to peak and the latency to the onset. For the functional analysis of locomotion, we painted the plantar surface of rat hindpaws with acrylic paint and allowed the rat walking along a corridor with white paper at the bottom. Footprints from operated and intact paws were analyzed by measuring the print length (PL), the distance between 1st and 5th (TS) or 2nd and 4th (IT) toes with a precision device. The three parameters were combined to obtain the Sciatic Functional Index (SFI)(de Medinaceli, 1988) which quantifies the changes in walking pattern (0 for uninjured; -100 for maximally impaired gait).

*Neuromuscular junction reinnervation analysis*

We cut the plantar interossei muscles into serial transverse sections (40 μm thick) using a cryotome and

preserved them at -20°C until use. The slides were incubated with chicken anti-Neuro Filament 200 (NF-200; 1:1000, Millipore) as described above and after several washes Cy3-conjugated secondary antibody was added. Finally, we incubated slices with α-Bungarotoxin labelling solution (Life technologies) following the manufacturer's protocol to reveal motor end plate machinery. Sequential microphotographs were taken covering all the plantar muscle at 20x. Only motor end plates with NF-200 co-labelling were counted as reinnervated.

**Conclusions**

Combination C1, comprising Acamprosate calcium and Ribavirin, showed an unexpected neuroprotective and neuroregenerative effect for motoneurons in both the tunicamycin-based *in vitro* model of ER stress and the *in vivo* model of peripheral nerve root avulsion. Furthermore, analysis of the neuroprotective effect demonstrated that C1 promotes motoneuron survival in vivo and presented supra-addictive effects in vitro. Besides, C1 reduces inflammatory-related states of the central nervous system after axonal injury and promotes the appearance of pro-regenerative neuronal phenotype. Lastly, the in silico predicted mechanism of action exerted by C1 over the pathological condition seem to be ascertain and increasing some cytoskeletal and pro-regenerative molecules, down-regulating some apoptotic molecules and increasing Sirt1 activity.

**Claims**

1. Combination comprising acamprosate or a pharmaceutically acceptable salt thereof, and ribavirin or a pharmaceutically acceptable salt thereof.

2. Combination according to claim 1, wherein the ratio of acamprosate or a pharmaceutically acceptable salt thereof, and ribavirin or a pharmaceutically acceptable salt thereof, is from 5000:1 to 1:1 by weight expressed as equivalent free acamprosate and equivalent free ribavirin.

3. Combination according to claim 2, wherein the ratio of acamprosate or a pharmaceutically acceptable salt thereof, and ribavirin or a pharmaceutically acceptable salt thereof, is from 50:1 to 30:1 by weight expressed as equivalent free acamprosate and equivalent free ribavirin.

4. Combination according to any one of the preceding claims wherein acamprosate is in the form of its calcium (2:1) salt and ribavirin is in the form of the free base.

5. Combination according to any one of the preceding claims wherein acamprosate or a pharmaceutically acceptable salt thereof, and ribavirin or a pharmaceutically acceptable salt thereof form part of the same composition.

6. Pharmaceutical composition comprising the combination according to any one of claims 1 to 5 and one or more pharmaceutically acceptable carriers.

7. Combination according to any one of claims 1 to 5 or pharmaceutical composition according to claim 6 for use in medicine.

8. Combination according to any one of claims 1 to 5 or pharmaceutical composition according to claim 6 for use in the treatment and/or prevention of neurological damage.

9. Combination or pharmaceutical composition for use according to claim 8, wherein the neurological damage is a condition or disease selected from the group consisting of peripheral nerve lesion, lesions of efferent/afferent fibers, lesions of the posterior root of the spinal cord, lesions or traumatic cranial neuropathy affecting cranial nerves, diseases of the motoneurons of the posterior root of the spinal cord or motor axons, diseases affecting lower motoneurons, acute and chronic inflammatory pain, neuropathic pain, neuritis, phantom limb pain, radiculopathy, traumatic nerve injury, inflammatory and neurodegenerative processes ensuing after the implantation of surgical prostheses.

10. Combination or pharmaceutical composition for use according to claim 9, wherein the lesions of efferent/afferent fibers are selected from the group consisting of polyneuropathies, nerve entrapments, nerve compression syndrome and nerve traumatism; wherein the lesions of the posterior root of the spinal cord are selected from the group consisting of radiculopathies, spinal disc herniations, or are caused by tumors and radiotherapy; wherein the cranial

nerve affected by the lesions or traumatic cranial neuropathy is selected from the group consisting of olfactory, oculomotor, trochlear, abducens, facial, vestibulocochlear, accessory and hypoglossal nerves, trigeminal nerve and ganglion, optic nerve and chiasm; wherein the disease of the motoneurons of the posterior root of the spinal cord is acute polyomelitis; wherein the disease of the motor axons is Guillain-Barré syndrome; and/or wherein disease affecting lower motoneurons is selected from the group consisting of peripheral neuropathies, amyotrophic lateral sclerosis and spinal muscular atrophy.

**11.** Combination or pharmaceutical composition for use according to claim 9, wherein the neurological damage is traumatic nerve injury.

**12.** Combination or pharmaceutical composition for use according to claim 11, wherein the traumatic nerve injury is peripheral nerve injury.

**13.** Kit comprising the combination according to any one of claims 1 to 4, wherein acamprosate or a pharmaceutically acceptable salt thereof and ribavirin or a pharmaceutically acceptable salt thereof are disposed in separate containers.

## Patentansprüche

**1.** Kombination, umfassend Acamprosat oder ein pharmazeutisch verträgliches Salz davon und Ribavirin oder ein pharmazeutisch verträgliches Salz davon.

**2.** Kombination nach Anspruch 1, wobei das Verhältnis von Acamprosat oder einem pharmazeutisch verträglichen Salz davon und Ribavirin oder einem pharmazeutisch verträglichen Salz davon von 5000:1 bis 1:1 Gewichtsanteile beträgt, ausgedrückt als äquivalentes freies Acamprosat und äquivalentes freies Ribavirin.

**3.** Kombination nach Anspruch 2, wobei das Verhältnis von Acamprosat oder einem pharmazeutisch verträglichen Salz davon und Ribavirin oder einem pharmazeutisch verträglichen Salz davon von 50:1 bis 30:1 Gewichtsanteile beträgt, ausgedrückt als äquivalentes freies Acamprosat und äquivalentes freies Ribavirin.

**4.** Kombination nach einem der vorhergehenden Ansprüche, wobei Acamprosat in der Form seines Calcium (2:1) Salzes vorliegt und Ribavirin in der Form der freien Base vorliegt.

**5.** Kombination nach einem der vorhergehenden Ansprüche, wobei Acamprosat oder ein pharmazeutisch verträgliches Salz davon und Ribavirin oder ein pharmazeutisch verträgliches Salz davon Bestandteile derselben Zusammensetzung sind.

**6.** Pharmazeutische Zusammensetzung, umfassend die Kombination nach einem der Ansprüche 1 bis 5 und einen oder mehrere pharmazeutisch verträgliche Träger.

**7.** Kombination nach einem der Ansprüche 1 bis 5 oder pharmazeutische Zusammensetzung nach Anspruch 6 zur Verwendung in der Medizin.

**8.** Kombination nach einem der Ansprüche 1 bis 5 oder pharmazeutische Zusammensetzung nach Anspruch 6 zur Verwendung bei der Behandlung und/oder Vorbeugung von neurologischen Schäden.

**9.** Kombination oder pharmazeutische Zusammensetzung zur Verwendung nach Anspruch 8, wobei der neurologische Schaden ein Zustand oder eine Krankheit ist, ausgewählt aus der Gruppe, bestehend aus peripherer Nervenläsion, Läsionen von efferenten/afferenten Fasern, Läsionen der hinteren Wurzel des Rückenmarks, Läsionen oder traumatische Schädelneuropathie, die die Hirnnerven betreffen, Erkrankungen der Motoneuronen der hinteren Rückenmarkswurzel oder der motorischen Axone, Erkrankungen der unteren Motoneuronen, akute und chronische entzündliche Schmerzen, neuropathische Schmerzen, Neuritis, Phantomschmerzen der Gliedmaßen, Radikulopathie, traumatische Nervenverletzung, entzündliche und neurodegenerative Prozesse, die nach der Implantation von chirurgischen Prothesen auftreten.

**10.** Kombination oder pharmazeutische Zusammensetzung zur Verwendung nach Anspruch 9, wobei die Läsionen von efferenten/afferenten Fasern ausgewählt sind aus der Gruppe, bestehend aus Polyneuropathien, Nerveneinklemmungen, Nervenkompressionssyndrom und Nerventrauma; wobei die Läsionen der hinteren Wurzel des Rückenmarks ausgewählt sind aus der Gruppe, bestehend aus Radikulopathien, Bandscheibenvorfällen oder verursacht durch Tumore und Strahlentherapie; wobei der von den Läsionen oder der traumatischen Hirnneuropathie betroffene Hirnnerv ausgewählt ist aus der Gruppe, bestehend aus Riech-, Augenbewegungs-, Trochlea-, Abduzen-, Gesichts-, Vestibulocochlear-, akzessorischen und hypoglossalen Nerven, Trigeminusnerv und Ganglion, Sehnerv und Chiasmus; wobei die Erkrankung der Motoneurone der hinteren Wurzel des Rückenmarks eine akute Polyomelitis ist; wobei die Erkrankung der motorischen Axone das Guillain-Barré-Syndrom ist; und/oder wobei die Erkrankung, die die unteren Motoneuronen betrifft, aus der Gruppe ausgewählt ist, die aus peripheren Neuropathien, amyotropher Lateralsklerose und spinaler Muskelatrophie besteht.

**11.** Kombination oder pharmazeutische Zusammensetzung zur Verwendung nach Anspruch 9, wobei der neurologische Schaden eine traumatische Nervenverletzung ist.

**12.** Kombination oder pharmazeutische Zusammensetzung zur Verwendung nach Anspruch 11, wobei die traumatische Nervenverletzung eine periphere Nervenverletzung ist.

**13.** Kit, umfassend die Kombination nach einem der Ansprüche 1 bis 4, wobei Acamprosat oder ein pharmazeutisch verträgliches Salz davon und Ribavirin oder ein pharmazeutisch verträgliches Salz davon in getrennten Behältern angeordnet sind.

## Revendications

**1.** Combinaison comprenant de l'acamprosate ou un sel pharmaceutiquement acceptable de celui-ci, et de la ribavirine ou un sel pharmaceutiquement acceptable de celle-ci.

**2.** Combinaison selon la revendication 1, dans laquelle le rapport entre l'acamprosate ou un sel pharmaceutiquement acceptable de celui-ci, et la ribavirine ou un sel pharmaceutiquement acceptable de celle-ci, est de 5000:1 à 1:1 en poids, exprimé en acamprosate libre équivalent et en ribavirine libre équivalente.

**3.** Combinaison selon la revendication 2, dans laquelle le rapport entre l'acamprosate ou un sel pharmaceutiquement acceptable de celui-ci, et la ribavirine ou un sel pharmaceutiquement acceptable de celle-ci, est compris entre 50:1 et 30:1 en poids exprimé en acamprosate libre équivalent et en ribavirine libre équivalente.

**4.** Combinaison selon l'une quelconque des revendications précédentes, dans laquelle l'acamprosate est sous la forme de son sel de calcium (2:1) et la ribavirine est sous la forme de la base libre.

**5.** Combinaison selon l'une quelconque des revendications précédentes, dans laquelle l'acamprosate ou un sel pharmaceutiquement acceptable de celui-ci, et la ribavirine ou un sel pharmaceutiquement acceptable de celle-ci font partie de la même composition.

**6.** Composition pharmaceutique comprenant la combinaison selon l'une quelconque des revendications 1 à 5 et un ou plusieurs véhicules pharmaceutiquement acceptables.

**7.** Combinaison selon l'une quelconque des revendications 1 à 5 ou composition pharmaceutique selon la revendication 6, pour l'utilisation comme medicament.

**8.** Combinaison selon l'une quelconque des revendications 1 à 5 ou composition pharmaceutique selon la revendication 6, pour l'utilisation dans le traitement et/ou la prévention de dommages neurologiques.

**9.** Combinaison ou composition pharmaceutique pour l'utilisation selon la revendication 8, dans laquelle le dommage neurologique est une affection ou maladie choisie dans le groupe consistant en la lésion nerveuse périphérique, les lésions de fibres efférentes/afférentes, les lésions de la racine postérieure de la moelle épinière, les lésions ou la neuropathie crânienne traumatique touchant les nerfs crâniens, les maladies des motoneurones de la racine postérieure de la moelle épinière ou des axones moteurs, les maladies affectant les motoneurones inférieurs, la douleur inflammatoire aiguë et chronique, la douleur neuropathique, la névrite, la douleur du membre fantôme, la radiculopathie, la lésion nerveuse traumatique, les processus inflammatoires et neurodégénératifs suite à l'implantation de prothèses chirurgicales.

**10.** Combinaison ou composition pharmaceutique pour l'utilisation selon la revendication 9, dans laquelle les lésions de fibres efférentes/afférentes sont choisies dans le groupe consistant en les polyneuropathies, les coincements nerveux, le syndrome de compression nerveuse et les traumatisme nerveux; dans laquelle les lésions de la racine postérieure de la moelle épinière sont choisies dans le groupe consistant en les radiculopathies, les hernies discales, ou sont causées par les tumeurs et la radiothérapie; dans laquelle le nerf crânien affecté par les lésions ou la neuropathie crânienne traumatique est choisi dans le groupe consistant en les nerfs olfactif, oculomoteur, trochléaire, abducens, facial, vestibulocochléaire, les nerfs accessoire et hypoglosse, le nerf trijumeau et le ganglion, le nerf optique et le chiasma; dans laquelle la maladie des motoneurones de la racine postérieure de la moelle épinière est une poliomyélite aiguë; dans laquelle la maladie des axones moteurs est le syndrome de Guillain-Barré; et/ou dans laquelle la maladie affectant les motoneurones inférieurs est choisie dans le groupe consistant en les neuropathies périphériques, la sclérose latérale amyotrophique et l'atrophie musculaire spinale.

**11.** Combinaison ou composition pharmaceutique pour l'utilisation selon la revendication 9, dans laquelle le dommage neurologique est une lésion nerveuse traumatique.

**12.** Combinaison ou composition pharmaceutique pour l'utilisation selon la revendication 11, dans laquelle la lésion nerveuse traumatique est une lésion nerveuse périphérique.

**13.** Kit comprenant la combinaison selon l'une quelconque des revendications 1 à 4, dans laquelle l'acamprosate ou un sel pharmaceutiquement acceptable de celui-ci et la ribavirine ou un sel pharmaceutiquement acceptable de celle-ci sont disposés dans des récipients séparés.

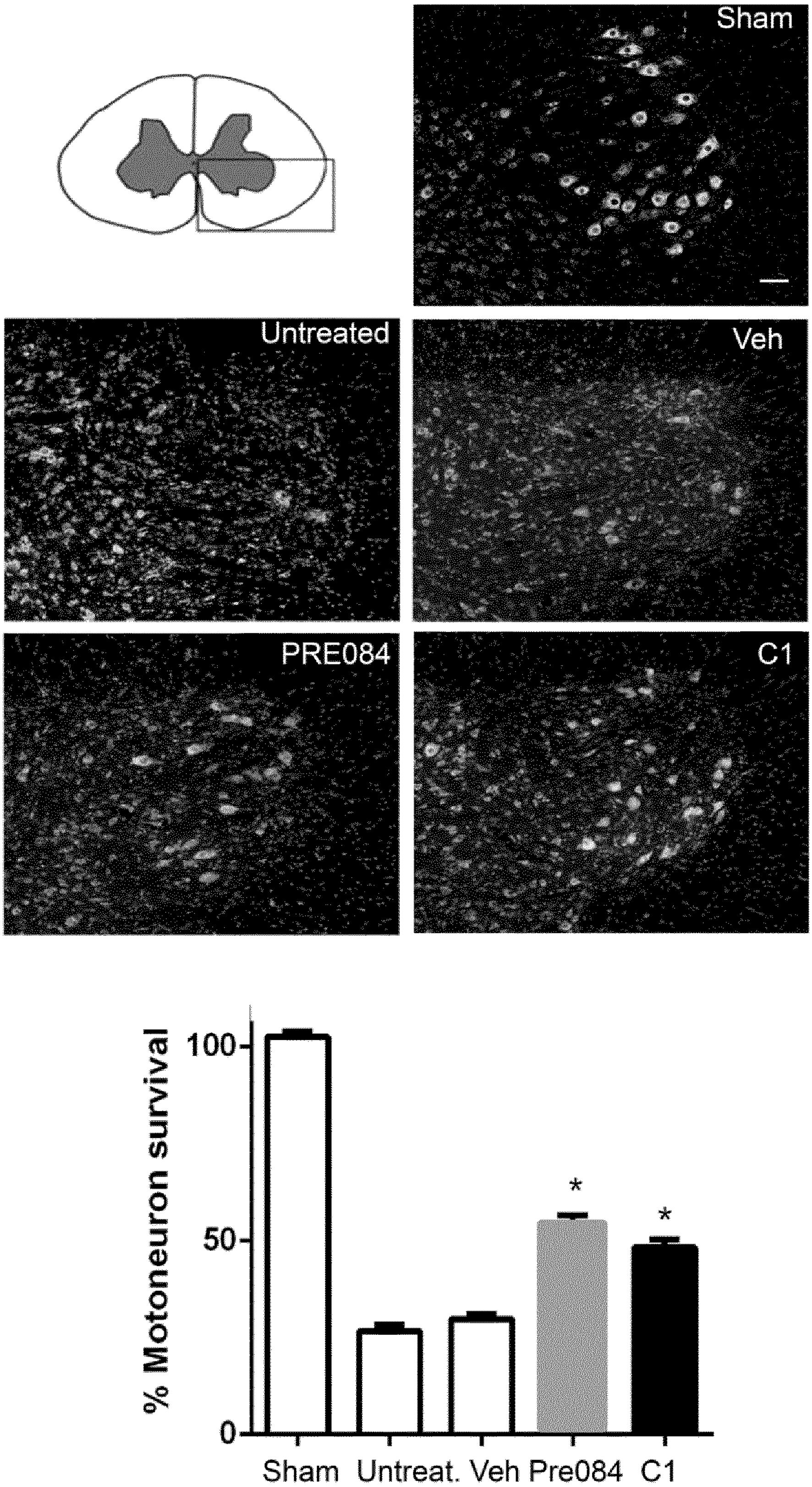
Sham
Untreated
Veh
PRE084
C1
% Motoneuron survival
100
50
0
*
*
Sham
Untreat.
Veh
Pre084
C1

FIG. 1

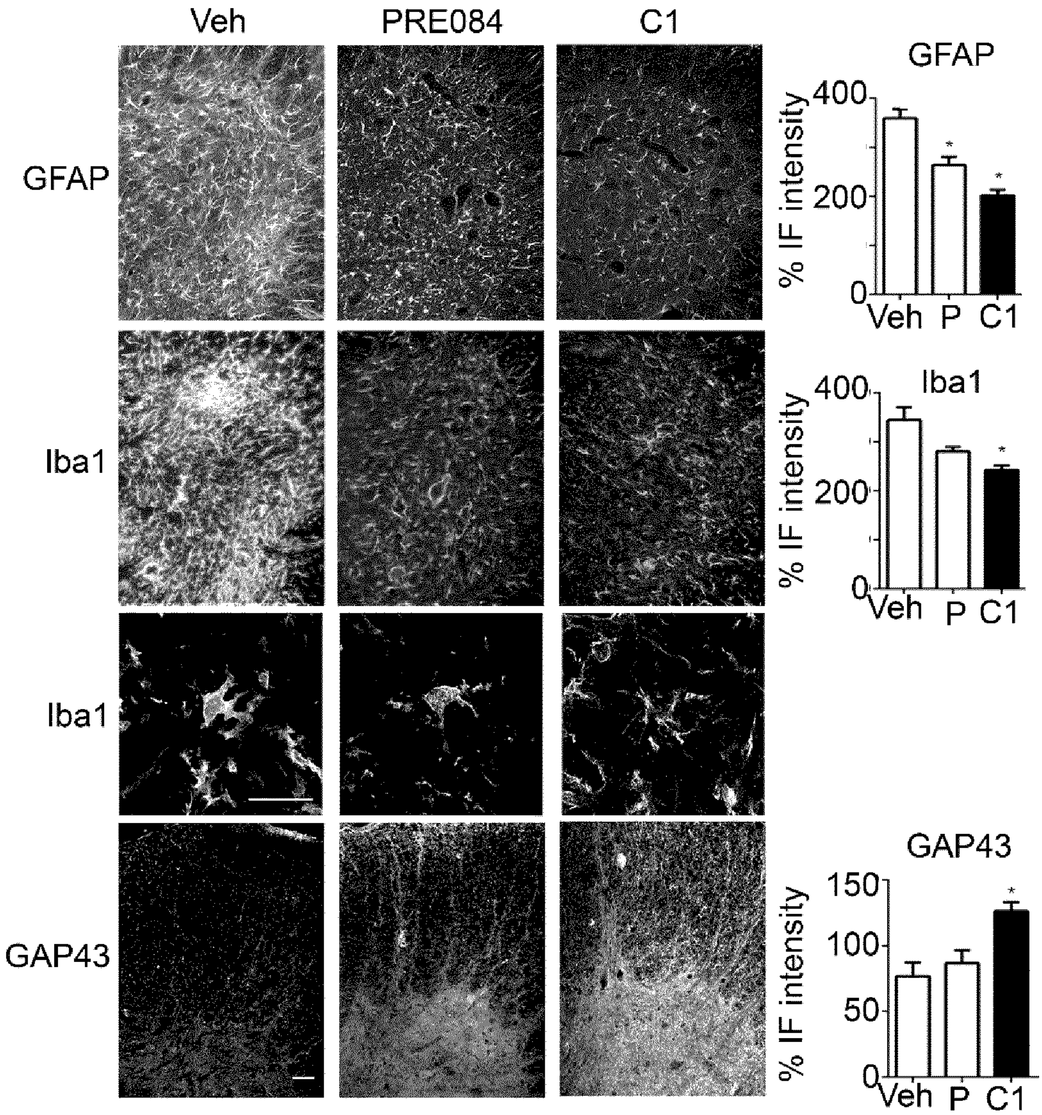
Veh
PRE084
C1
GFAP
Iba1
Iba1
GAP43
GFAP
400
200
0
% IF intensity
Veh P C1
Iba1
400
200
0
% IF intensity
Veh P C1
GAP43
150
100
50
0
% IF intensity
Veh P C1

**FIG. 2**

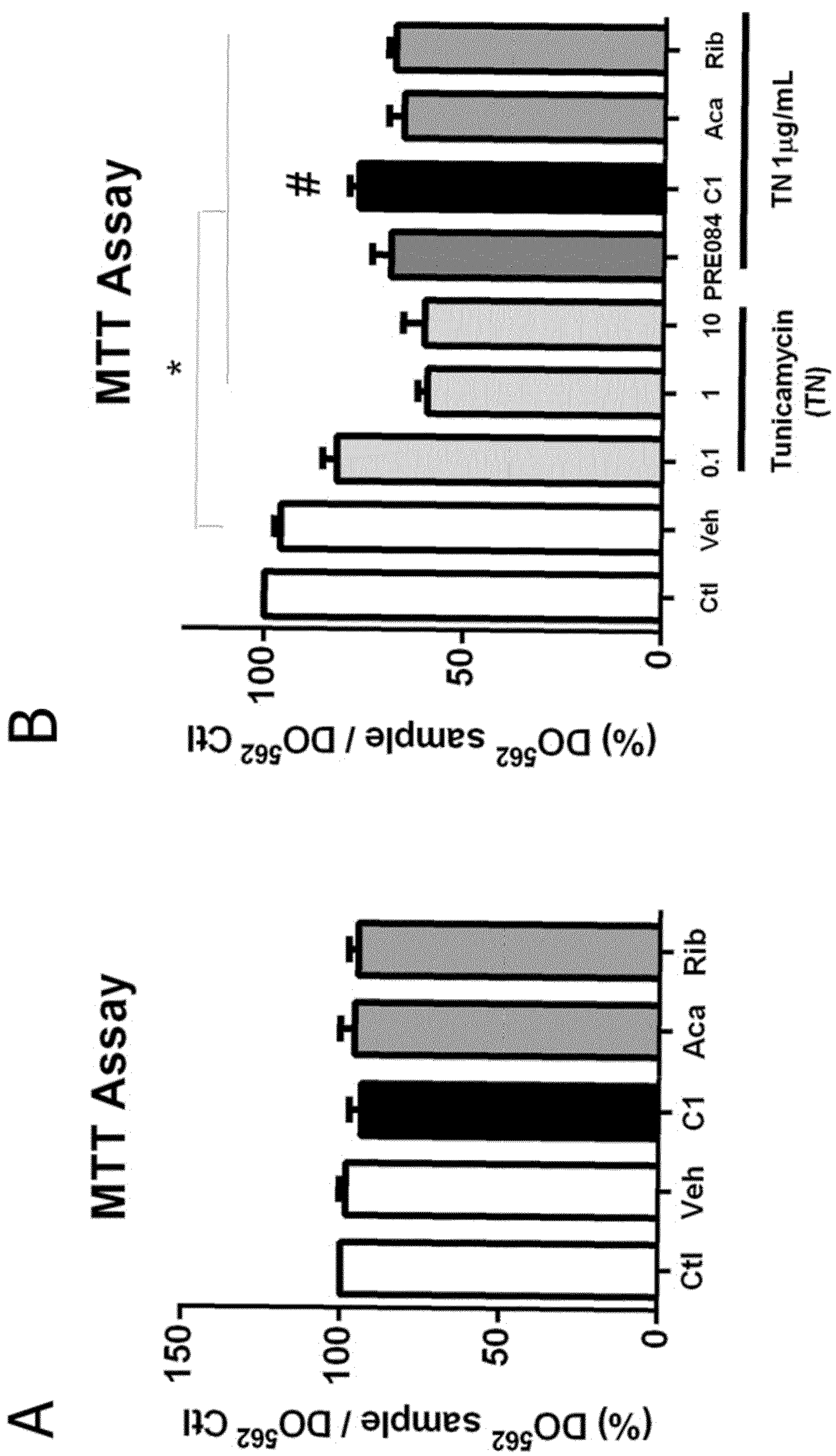
A
MTT Assay
(%) DO562 sample / DO562 Ctl
150
100
50
0
Ctl
Veh
C1
Aca
Rib
B
MTT Assay
(%) DO562 sample / DO562 Ctl
100
50
0
*
#
Ctl
Veh
0.1
1
10
PRE084
C1
Aca
Rib
Tunicamycin
(TN)
TN 1μg/mL

FIG. 3

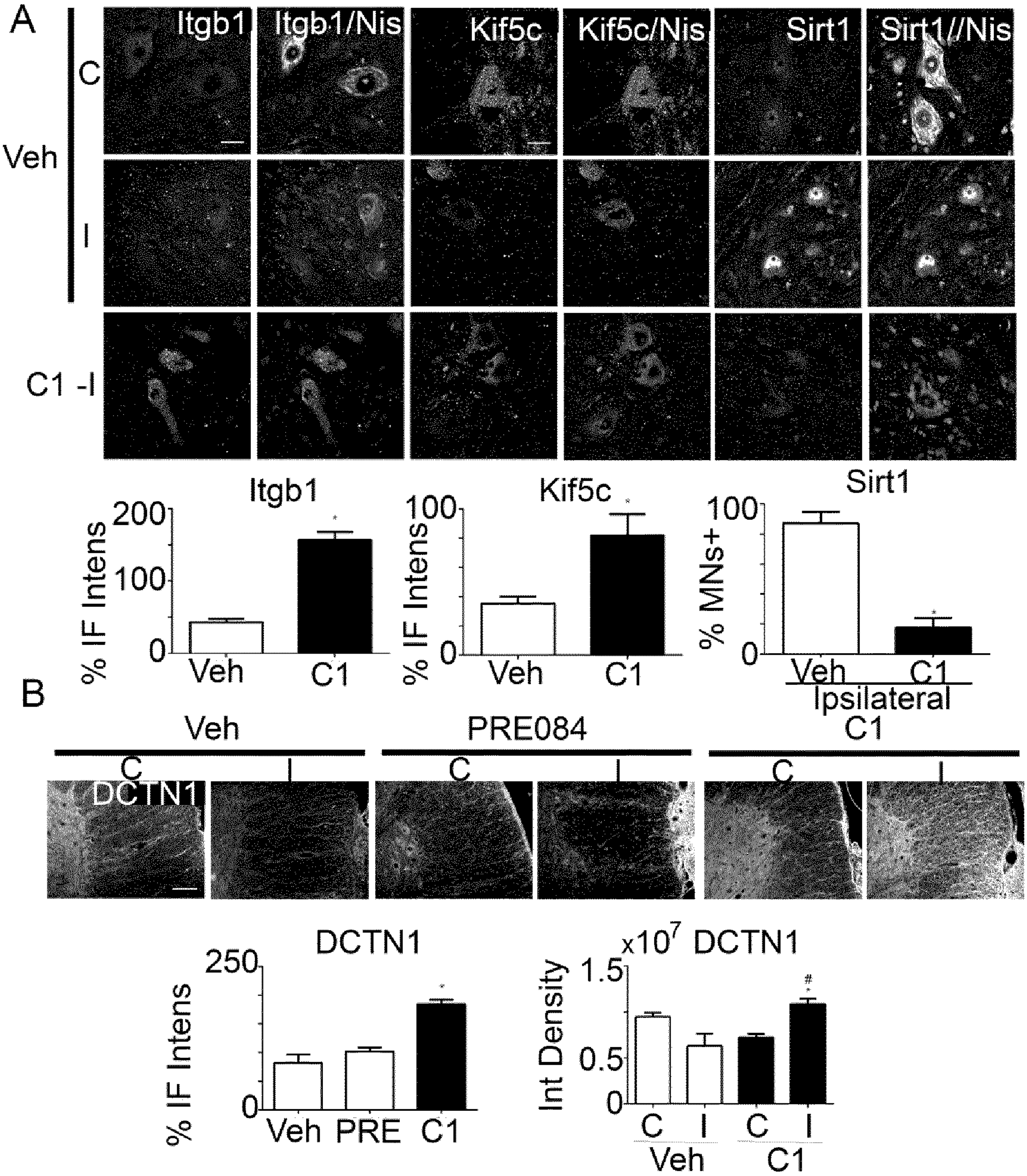
A
Veh
C
I
C1 -I
Itgb1
Itgb1/Nis
Kif5c
Kif5c/Nis
Sirt1
Sirt1//Nis
Itgb1
% IF Intens
200
100
0
Veh
C1
Kif5c
% IF Intens
100
0
Veh
C1
Sirt1
% MNs+
100
0
Veh
C1
Ipsilateral
B
Veh
PRE084
C1
C
I
C
I
C
I
DCTN1
DCTN1
% IF Intens
250
0
Veh
PRE
C1
x10^7 DCTN1
Int Density
1.5
0.5
0
C
I
C
I
Veh
C1

FIG. 4

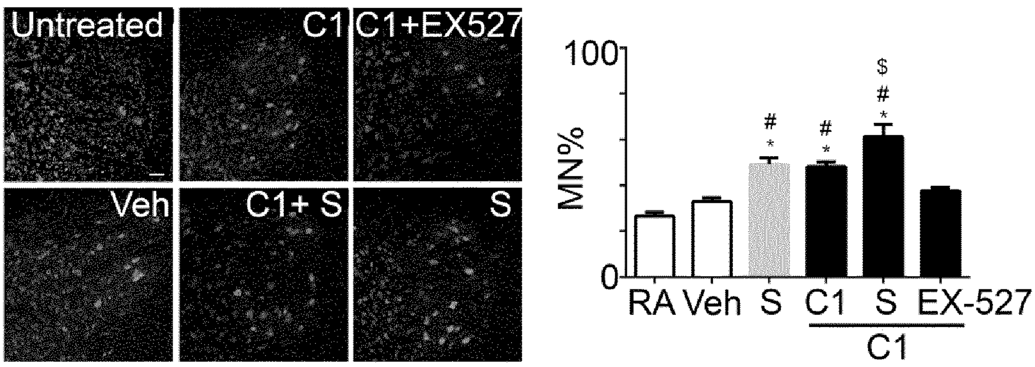


FIG. 5

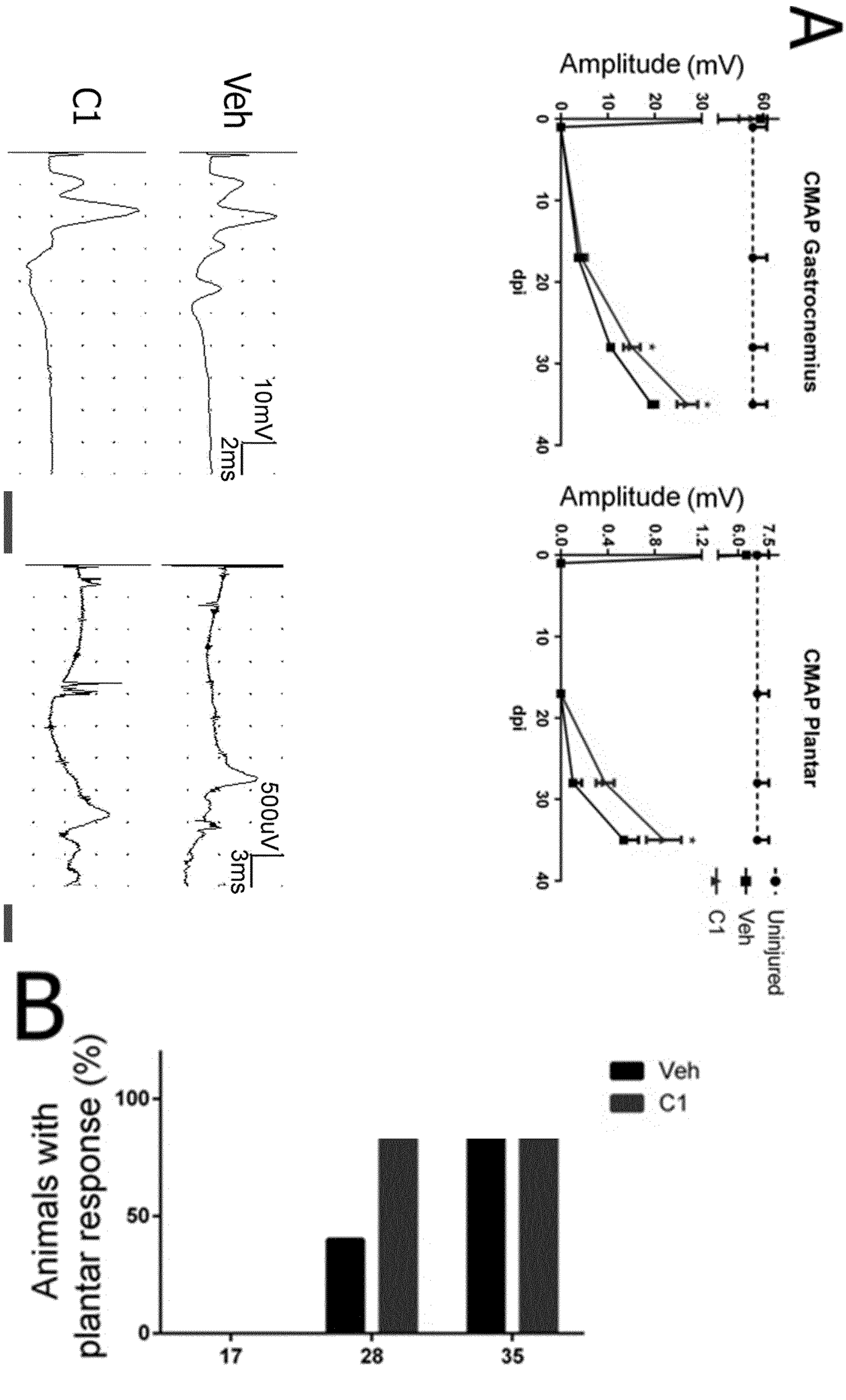
A
CMAP Gastrocnemius
Amplitude (mV)
dpi
CMAP Plantar
Amplitude (mV)
dpi
Uninjured
Veh
C1
Veh
C1
10mV
2ms
500uV
3ms
B
Animals with plantar response (%)
Veh
C1
17
28
35

FIG. 6

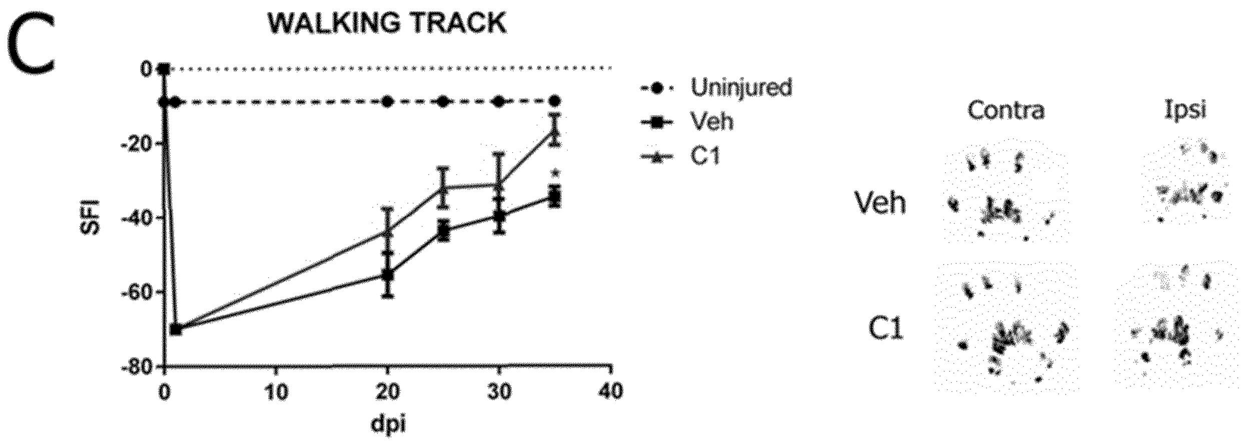
C
WALKING TRACK
SFI
dpi
0
-20
-40
-60
-80
0
10
20
30
40
Uninjured
Veh
C1
*
Contra
Ipsi
Veh
C1

FIG. 6

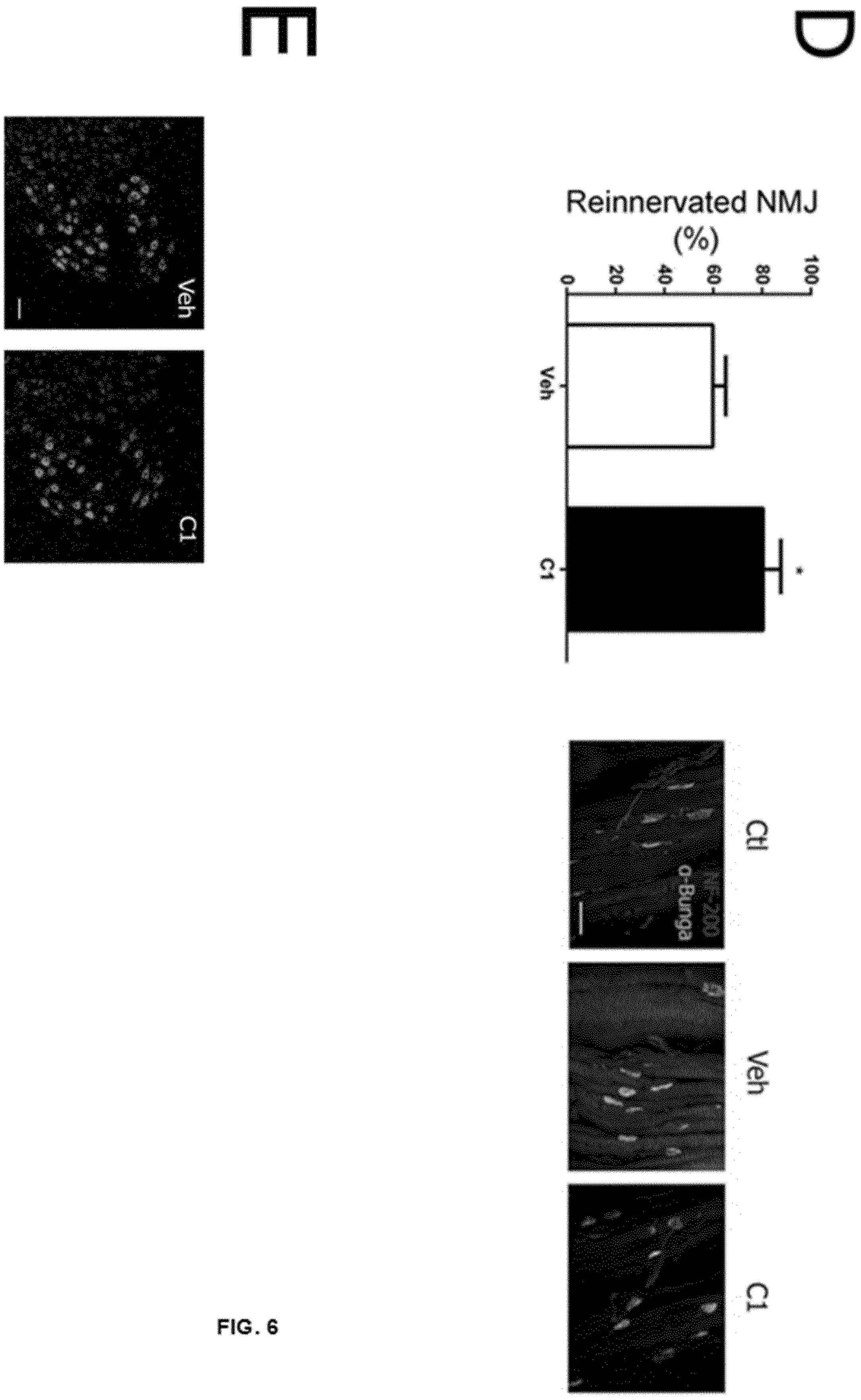
D
E
Reinnervated NMJ (%)
0
20
40
60
80
100
Veh
C1
*
Ctl
Veh
C1
NF-200
α-Bunga
Veh
C1

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2010216734 A


### Non-patent literature cited in the description


- **VALERO-CABRÉ A.** Peripheral and spinal motor reorganization after nerve injury and repair. *J Neurotrauma,* 2004
- **PENAS C.** Autophagy, and BiP level decrease are early key events in retrograde degeneration of motoneurons. *Cell Death Differ.,* 2011
- **CHEW DJ.** The effects of minocycline or riluzole treatment on spinal root avulsion-induced pain in adult rats. *J Pain.,* 2014
- **PENAS, C. et al.** Sigma Receptor Agonist 2-(4-Morpholinethyl)1 Phenylcyclohexanecarboxylate (Pre084) Increases GDNF and BiP Expression and Promotes Neuroprotection after Root Avulsion Injury. *J. Neurotrauma,* 2011, vol. 28, 831-840
- **NAVARRO X.** Neural plasticity after peripheral nerve injury and regeneration. *Prog Neurobiol.,* 2007
- **MONTAGUE K.** Endoplasmic reticulum stress in spinal and bulbar muscular atrophy: a potential target for therapy. *Brain,* 2014
- **HETZ C.** Disturbance of endoplasmic reticulum proteostasis in neurodegenerative diseases. *Nat Rev Neurosci,* 2014
- **REAGAN-SHAW S.** Dose translation from animal to human studies revisited. *FASEB J,* 2007, vol. 22, 659-661
- Methods in Cell Biology. Academic Press, 1976, vol. XIV, 33
- **BROOKS BANK et al.** *BIOGRID38, MINT,* 2003
- **PENAS C.** Cytoskeletal and activity-related changes in spinal motoneurons after root avulsion. *Journal of Neurotrauma,* 2009
- **PENAS C.** Sigma receptor agonist 2-(4-morpholinethyl)1 phenylcyclohexanecarboxylate (Pre084) increases GDNF and BiP expression and promotes neuroprotection after root avulsion injury. *J Neurotrauma,* 2011